(19) Europäisches Patentamt / European Patent Office / Office européen des brevets

(11) EP 3 135 190 A1

(12) EUROPEAN PATENT APPLICATION

(43) Date of publication:
01.03.2017 Bulletin 2017/09

(51) Int Cl.:
A61B 5/00 $^{(2006.01)}$        A61B 8/00 $^{(2006.01)}$

(21) Application number: 16185288.4

(22) Date of filing: 23.08.2016

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD

(30) Priority: 24.08.2015  JP 2015164669
04.09.2015  JP 2015175013

(71) Applicant: Canon Kabushiki Kaisha
Tokyo 146-8501 (JP)

(72) Inventor: KANDORI, Atsushi
Ohta-ku, Tokyo 146-8501 (JP)

(74) Representative: Williamson, Brian
Canon Europe Ltd
European Patent Department
3 The Square
Stockley Park
Uxbridge, Middlesex UB11 1ET (GB)

(54) **ACOUSTIC WAVE PROBE, ACOUSTIC WAVE TRANSDUCER UNIT, AND OBJECT INFORMATION ACQUISITION APPARATUS**

(57)    An acoustic wave probe includes a support member including a plurality of through holes and a concave portion which is concave facing an object disposed in a measurement position at the time of measurement, and an acoustic wave transducer unit including at least a transducer. The acoustic wave transducer unit is mounted in the through hole facing approximately a center of curvature of the concave portion, and a thickness of the acoustic wave transducer unit is thinner on a side of the center of curvature.

**FIG.1B**

**FIG.1D**

EP 3 135 190 A1

**Description**

BACKGROUND OF THE INVENTION

Field of the Invention

The present invention relates to an acoustic wave probe capable of receiving an acoustic wave, such as a photoacoustic wave due to the photoacoustic effect, (hereinafter, there are cases where referred to as ultrasound), an acoustic wave transducer unit, and an object information acquisition apparatus using the acoustic wave probe or the acoustic wave transducer unit.

Description of the Related Art

U.S. Patent Publication No. 2011/0306865 discusses a measurement system which receives a photoacoustic wave (typically ultrasound) generated at a measurement object in an object due to the photoacoustic effect when the object is irradiated with light, using an ultrasonic probe having a hemispherical shape. The ultrasonic probe with a hemispherical shape includes a plurality of ultrasonic transducer elements arranged on its hemispherical surface.

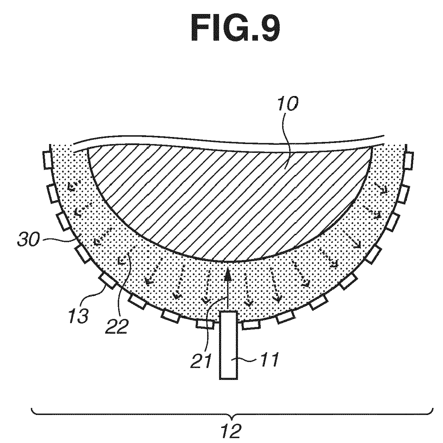

The measurement system will be described with reference to Fig. 9. In Fig. 9, there are provided the measurement system and an object 10. The measurement system includes a light source 11, an ultrasonic probe 12, an ultrasonic transducer 13, light 21, a photoacoustic wave 22, and a medium 30 (acoustic matching material). The ultrasonic probe 12 has a hemispherical shape, and includes a plurality of the ultrasonic transducers 13 and the light source 11. At the time of measurement, the object 10 is partly surrounded by the hemispherical ultrasonic probe 12, and a space between the object 10 and the ultrasonic probe 12 is filled with the medium 30. The object 10 is irradiated with the light 21 from the light source 11. The photoacoustic wave 22 is generated in the object 10, and received by the ultrasonic transducers 13 in the ultrasonic probe 12. Imaging of the object is executed based on the received signal.

To receive the photoacoustic wave from the object, the ultrasonic transducers need to be arranged at predetermined dispersed positions on the hemispherical surface. In U.S. Patent Publication No. 2011/0306865, however, the arrangement structure of the probe (transducer) on a cup-shaped container is not specifically described. In such a structure, if a sensor surface of the ultrasonic transducer comes in contact with the cup-shaped container at the time of installation, there is a possibility that characteristics of the ultrasonic transducer change. Therefore, the present invention is directed to providing an acoustic wave probe and so forth in which deterioration of characteristics of the transducer due to its installation is unlikely to occur.

Further, to receive a photoacoustic wave from the object, the ultrasonic transducers need to be arranged at predetermined dispersed positions on the hemispherical surface. In a case where ultrasonic transducers are supported by a support member in a manner as disclosed in U.S. Patent Publication No. 2011/0306865, the ultrasonic transducers can be readily arranged at the predetermined positions. However, the inventors found an issue. That is, in a case where ultrasonic transducers are disposed on a hemispherical support member and water is used as a medium, it is likely that a turbulent flow is generated and temperature distribution of the medium becomes uneven in association with the turbulent flow. As a result, noises may appear in an image of the object.

Therefore, the present invention is further directed to providing a photoacoustic wave probe of which structure is simple and with which a turbulent flow of acoustic matching material and unevenness of temperature distribution of the acoustic matching material in association with a turbulent flow are unlikely to occur, and a photoacoustic apparatus provided with the photoacoustic wave probe.

SUMMARY OF THE INVENTION

According to an aspect of the present invention, there is provided an acoustic wave probe as specified in claims 1 to 12. According to a second aspect of the present invention, there is provided acoustic wave transducer means as specified in claims 13 and 14. According to a third aspect of the present invention, there is provided an object information acquisition apparatus as specified in claim 15. According to a fourth aspect of the present invention, there is provided an object information acquisition apparatus as specified in claim 16. According to a fifth aspect of the present invention, there is provided a photoacoustic wave probe as specified in claims 17 to 19.

Further features of the present invention will become apparent from the following description of exemplary embodiments with reference to the attached drawings.

BRIEF DESCRIPTION OF THE DRAWINGS

Figs. 1A and 1B are diagrams each illustrating an acoustic wave probe according to a first exemplary embodiment. Fig. 1C is a diagram illustrating an acoustic wave transducer unit according to the first exemplary embodiment. Fig. 1D is a cross sectional view of the acoustic wave probe according to the first exemplary embodiment.

Fig. 2A is a diagram illustrating an acoustic wave transducer unit according to a second exemplary embodiment. Figs. 2B and 2C are cross sectional views each illustrating an acoustic wave probe according to the second embodiment.

Fig. 3A is a diagram illustrating an acoustic wave transducer unit according to a third exemplary embodiment. Figs. 3B and 3C are cross sectional views each illustrating an acoustic wave probe according to the third exemplary embodiment. Fig. 3D is a diagram illustrating the acoustic transducer unit viewed in a radial direction from the center of the acoustic wave probe according to the third exemplary embodiment.

Fig. 4A is a diagram illustrating an acoustic wave transducer unit according to a fourth exemplary embodiment. Fig. 4B is a cross sectional view illustrating an acoustic wave probe according to the fourth exemplary embodiment.

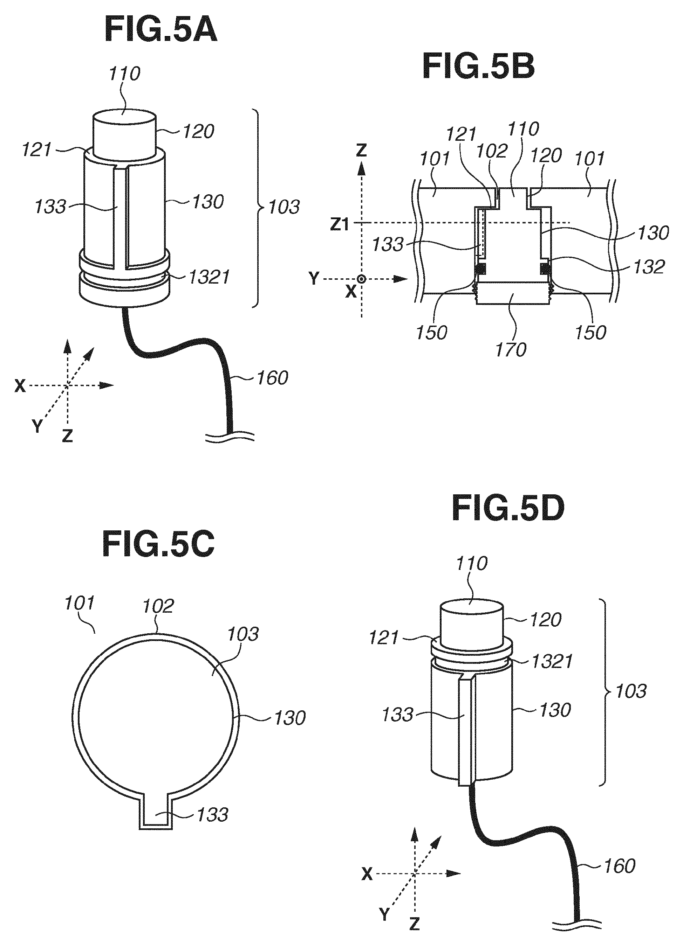

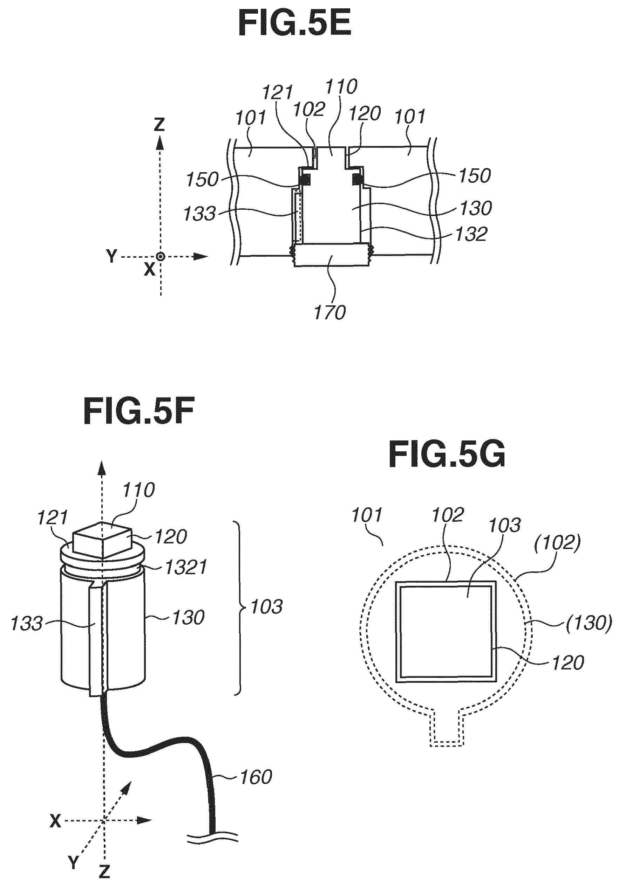

Fig. 5A is a diagram illustrating an acoustic wave transducer unit. Fig. 5B is a cross sectional view illustrating an acoustic wave probe. Fig. 5C is a transverse cross sectional view illustrating the acoustic wave transducer unit according to the fifth exemplary embodiment. Fig. 5D is a diagram illustrating an acoustic wave transducer unit of another example according to the fifth exemplary embodiment. Fig. 5E is a cross sectional view illustrating an acoustic wave probe of another example according to the fifth exemplary embodiment. Fig. 5F is a diagram illustrating an acoustic wave transducer unit of yet another example according to the fifth exemplary embodiment. Fig. 5G is a diagram illustrating the acoustic transducer unit of yet another example according to the fifth exemplary embodiment, viewed in a radial direction from the center of a hemisphere of the acoustic wave probe.

Fig. 6A is a diagram illustrating an acoustic wave transducer unit according to the sixth exemplary embodiment. Fig. 6B is a diagram illustrating a cross section of an acoustic wave probe according to the sixth exemplary embodiment. Fig. 6C is a transverse cross sectional view illustrating the acoustic wave transducer unit according to the sixth exemplary embodiment. Fig. 6D is a diagram illustrating an acoustic wave transducer unit of another example according to the sixth exemplary embodiment.

Fig. 7 is a diagram illustrating a schematic view of an object information acquisition apparatus according to a seventh exemplary embodiment.

Fig. 8 is a diagram illustrating a schematic view of an object information acquisition apparatus according to an eighth exemplary embodiment.

Fig. 9 is a diagram illustrating a schematic view of an acoustic wave probe.

Fig. 10A is a perspective view illustrating a photoacoustic wave probe according to a ninth exemplary embodiment. Fig. 10B is an A-A' cross sectional view. Fig. 10C is a perspective view illustrating an ultrasonic transducer arranged in the photoacoustic wave probe. Fig. 10D is an A-A' cross sectional view of a photoacoustic wave probe of another example. Fig. 10E is a perspective view of an ultrasonic transducer of another example.

Fig. 11A is a schematic cross sectional view illustrating a capacitive ultrasonic transducer (CMUT) according to the ninth exemplary embodiment, and Fig. 11B is a schematic cross sectional view illustrating a CMUT having another structure.

Fig. 12A is a cross sectional view illustrating an ultrasonic transducer according to a tenth exemplary embodiment. Fig. 12B is a circuit diagram illustrating a circuit provided in the ultrasonic transducer.

Figs. 13A, 13B, 13C, and 13D are circuit diagrams each illustrating an example of a circuit provided in an ultrasonic transducer according to an eleventh exemplary embodiment.

Fig. 14 is a cross sectional view illustrating an ultrasonic transducer according to a twelfth exemplary embodiment.

Fig. 15A is a perspective view illustrating an ultrasonic transducer according to a thirteenth exemplary embodiment. Fig. 15B is a cross sectional view illustrating the ultrasonic transducer.

Fig. 16 is a schematic cross sectional view illustrating a transducer unit according to a fourteenth exemplary embodiment.

Fig. 17A is a schematic cross sectional view illustrating a transducer unit according to a fifteenth exemplary embodiment. Fig. 17B is a schematic cross sectional view illustrating a transducer unit having another structure according to the fifteenth exemplary embodiment.

Fig. 18 is a schematic cross sectional view illustrating a transducer unit according to a sixteenth exemplary embodiment.

Figs. 19A and 19B are transverse cross sectional views each illustrating a transducer unit according to a seventeenth exemplary embodiment. Fig. 19C is a diagram illustrating a transducer unit having another structure.

Fig. 20A is a top schematic view illustrating a transducer unit according to an eighteenth exemplary embodiment. Fig. 20B is a cross sectional view illustrating the transducer unit. Fig. 20C is a diagram illustrating a circuit in a photoacoustic wave probe according to the eighteenth exemplary embodiment.

Fig. 21A is a schematic view illustrating a transducer according to a nineteenth exemplary embodiment. Fig. 21B is a circuit diagram illustrating an applied voltage adjusting circuit.

Fig. 22 is a schematic view illustrating a transducer according to a twentieth exemplary embodiment.

Fig. 23A is a schematic view illustrating a transducer according to a twenty-first exemplary embodiment. Fig. 23B is a circuit diagram illustrating a driving detecting circuit.

Fig. 24A is a schematic view illustrating a transducer according to a twenty-second exemplary embodiment. Fig. 24B is a circuit diagram illustrating a driving detecting circuit.

Fig. 25A is a schematic view illustrating a transducer according to a twenty-third exemplary embodiment.

Fig. 25B is a circuit diagram illustrating a driving detecting circuit according to the twenty-third exemplary embodiment. Fig. 25C is a circuit diagram illustrating a driving detecting circuit with another structure according to the twenty-third exemplary embodiment. Fig. 25D is a circuit diagram illustrating a driving detecting circuit with yet another structure according to the twenty-third exemplary embodiment. Fig. 26 is a schematic cross sectional view illustrating a photoacoustic apparatus according to a twenty-fourth exemplary embodiment.

Fig. 27 is a schematic cross sectional view illustrating a photoacoustic apparatus according to a twenty-fifth exemplary embodiment.

DESCRIPTION OF THE EMBODIMENTS

In exemplary embodiments described below, an acoustic wave probe includes a support member having a concave portion and an acoustic wave transducer unit including one or more transducers. The acoustic wave transducer unit is mounted in a through hole of the support member so as to face approximately a center of curvature of the concave portion, and the acoustic wave transducer unit is formed thinner on a side of the center of curvature of the concave portion. For example, in a support member with a hemispherical surface, an acoustic wave transducer unit is inserted and fixed in a through hole so as to face a center of the hemisphere. For example, the acoustic wave transducer unit includes a leading edge portion with a columnar shape (such as cylindrical shape and prismatic shape) and a columnar housing portion, and a plurality of ultrasonic transducers are arranged on a surface of the leading edge portion. The thickness of the leading edge portion is thinner than the thickness of the housing portion. The thickness of the housing portion may slightly vary. Typically, the cross sectional area of a leading edge portion of the acoustic wave probe (an end portion inside the hemispherical shape) is minimum, and the cross sectional area of a root portion of the acoustic wave probe (an end portion outside the hemispherical shape) is maximum.

Hereinafter, the present exemplary embodiments will be described. Although the present exemplary embodiments are described referring to a photoacoustic diagnosis apparatus, an ultrasonic diagnosis apparatus without a light source can also be used. This invention is not limited to the exemplary embodiments described below.

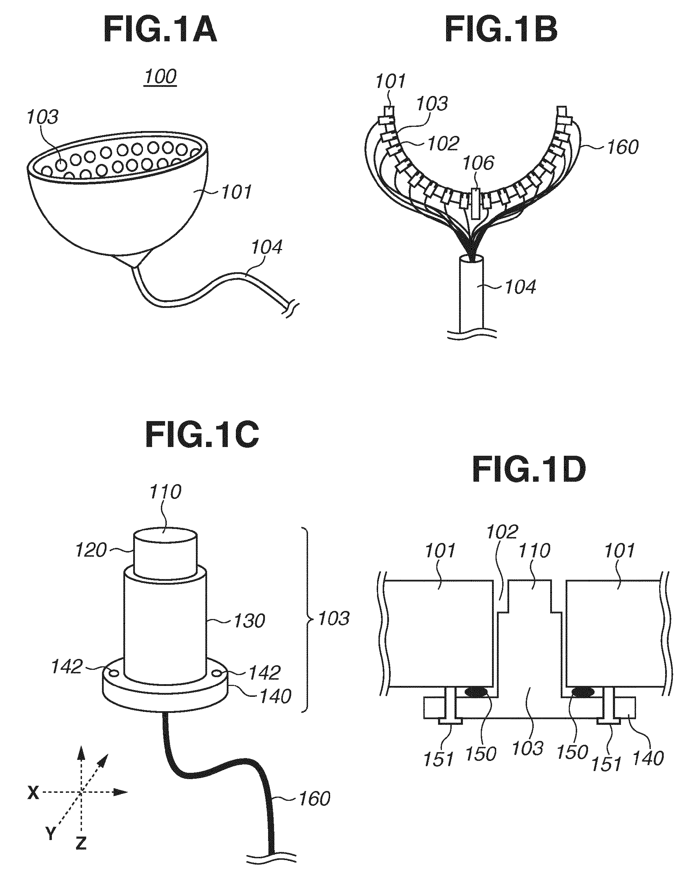

With reference to Figs. 1A to 1D, a photoacoustic wave probe (ultrasonic probe or acoustic wave probe) 100 according to a first exemplary embodiment will be described. The photoacoustic wave probe 100 includes a hemispherical support member 101 that is a probe housing, a through hole 102, an acoustic wave transducer unit (also referred to an ultrasonic transducer unit or simply a unit) 103, a cable bundle 104 of cables 160 each connected from a different one of the acoustic wave transducer units 103, a light source 106, and an ultrasonic transducer (also referred to simply as a transducer) 110. Fig. 1A is a schematic perspective view illustrating an external appearance of the photoacoustic wave probe 100 according to the present exemplary embodiment. Fig. 1B is a schematic cross sectional view illustrating the photoacoustic wave probe 100. Compared to the unit 103 illustrated in Fig. 1C and Fig. 1D, the unit 103 in Fig. 1B is illustrated in an abbreviated manner. Fig. 1C is a schematic perspective view illustrating the acoustic wave transducer unit 103. Fig. 1D is a schematic cross sectional view taken along an X-Z plane illustrating the unit 103 shown in Fig. 1C installed in the support member 101. In Fig. 1C and Fig. 1D, the cable 160 is omitted.

In the hemispherical support member 101 (approximately spherical), a plurality of through holes 102 are formed at positions corresponding to where the units 103 are respectively disposed. The acoustic wave transducer unit 103 has an outward form of an approximately cylindrical shape corresponding to a shape of the through hole 102. Each unit 103 is inserted and fixed in a different one of the through holes 102. The unit 103 includes one or more ultrasonic transducers 110 (individual transducers are not shown) disposed on a leading edge portion 120. The transducer 110 is arranged facing a location near a center of the hemisphere of the support member 101. In the present exemplary embodiment, a transverse cross sectional shape of the through hole 102 is a perfect circle, and one or more ultrasonic transducers 110 are disposed on a central part of the leading edge portion 120. Though it depends on cases, each unit 103 normally includes a number of the ultrasonic transducers 110 approximately from a hundred to a thousand. A plurality of the cables 160 of the units 103 are bundled up to a single cable bundle 104, and connected to a connector (not shown) which is connected to an external apparatus. Thus, the acoustic wave transducer unit includes the transducer (such as a capacitive electromechanical converter) disposed on its leading edge portion such that the transducer can perform at least one of conversion of an acoustic wave to a reception signal and conversion of a transmission signal to an acoustic wave. An outside diameter of the leading edge portion of the unit is, for example, approximately 10 mm. In a body (the housing portion) of the unit, flexible printed wiring, a receiving preamplifier, and the like are arranged.

The light source 106 is arranged in a central part of the hemispherical support member 101. The light source 106 can be any of what can emit light, such as a solid laser, a gas laser, a semiconductor laser, and a light emitting diode (LED). Light can be guided from a light emitting portion disposed outside, using an optical fiber. In the present exemplary embodiment, the light source 106 is arranged in the central part of the hemispherical support member 101, but the structure is not limited thereto. One or more light sources 106 can be arranged at any region of the hemispherical support member 101 where no acoustic wave transducer unit 103 is disposed.

According to the present exemplary embodiment, the acoustic wave transducer unit 103 is provided separately from the support member 101 and mounted in the through hole 102 of the support member 101. Therefore, the support member 101 can have a very simple structure having apertures formed in a concave member. Further, the support member 101 and the acoustic wave transducer unit 103 are separate from each other, so that only an operation-tested unit 103 can be selected and used. Accordingly, yield of the acoustic wave probe 100 can be readily improved. Further, in a case where the unit 103 is broken down, replacement can be easily executed. Moreover, an ultrasonic probe 100 with a different sensor interval can be readily prepared by changing only positions of the through holes 102 in the support member 101. Similarly, a hemispherical probe with a different radius can be provided by preparing the support member 101 with a different radius and using the same acoustic wave transducer unit 103.

Any of the ultrasonic transducer 110 capable of receiving and transmitting ultrasound can be used. A piezo-type (PZT type) ultrasonic probe capable of being used by connecting to an object information acquisition apparatus can be used. Further, a polyvinylidene fluoride type (PVDF type) ultrasonic probe, a capacitive ultrasonic transducer (CMUT), and the like can be used. The CMUT is particularly preferable since it can receive ultrasound in a broad band region with high sensitivity.

In the transducer unit 103 according to the present exemplary embodiment, the leading edge portion 120 on which the ultrasonic transducer 110 is disposed is thinner than the rest of the portion (also referred to as the housing portion 130). Accordingly, when the unit 103 is inserted and fixed in the through hole 102 of the support member 101, it is possible to prevent the ultrasonic transducer 110 on the leading edge portion 120 from coming in contact with a side face of the through hole 102 in the support member 101. Therefore, assemblage can be achieved with little deterioration of performance of the ultrasonic transducer 110.

As shown in Fig. 1C and Fig. 1D, a protruding plate portion (a flange portion) 140 is formed at an end portion of the housing portion 130 in the acoustic wave transducer unit 103 (i.e., an end portion opposite to an end portion where the ultrasonic transducer 110 is disposed). The protruding plate portion 140 is larger than the through hole 102. As shown in Fig. 1D, the shape of a longitudinal cross section of the end portion, of the unit 103, opposite to the leading edge portion 120 is T-shaped. The protruding plate portion 140 has an aperture 142 into which a screw is inserted, and the unit 103 can be fixed to the support member 101 with a screw 151 through the protruding plate portion 140. More specifically, the unit 103 is fixed in a manner such that an O-ring 150 is interposed and crushed a little between the protruding plate portion 140 and the support member 101. A gap between the acoustic wave transducer unit 103 and the support member 101 is sealed by the O-ring 150. Hence, an acoustic matching material, such as water and ultrasound gel, put on an object side is prevented from leaking out to a rear side (an outer surface of the hemispherical support member 101) of the support member 101, and remains kept inside the support member 101. In such a manner, the sealing member, such as an O-ring, is interposed between the acoustic wave transducer unit and the support member to prevent leakage of the acoustic matching material.

According to the present exemplary embodiment, the leading edge portion 120 of the unit 103 is smaller than the housing portion 130 (however, the housing portion 130 can include a portion thinner than the leading edge portion 120). Therefore, when the unit 103 is inserted in the through hole 102, contact between the leading edge portion 120 and the inside of the through hole 102 is unlikely to occur. According to such a photoacoustic wave probe 100, at the time of assemblage of the support member 101 and the unit 103, deterioration of performance of the ultrasonic transducer 110 is unlikely to occur. Further, since assemblage is conducted such that each unit 103 is separately inserted into the support member 101, it is possible to select a good product of the acoustic wave transducer unit 103 and mount it to the support member 101. Hence, a probe with receiving elements having uniform characteristics can be provided. Further, since the sealing member, such as the O-ring 150, is employed, the unit 103 can be brought into accurate contact with the support member 101, and the matching medium can be certainly kept in a space between the object and the photoacoustic wave probe 100. Additionally, since bubbles and the like are hardly generated in the matching medium provided in a concave side of the photoacoustic wave probe 100, deterioration of signals due to the bubbles and the like is unlikely to occur. Hence, the photoacoustic wave can be received with high quality. Furthermore, since the medium, such as water, hardly leaks out, deterioration of reliability of electric components due to the leakage is unlikely to occur. Thus, a probe with high reliability can be provided.

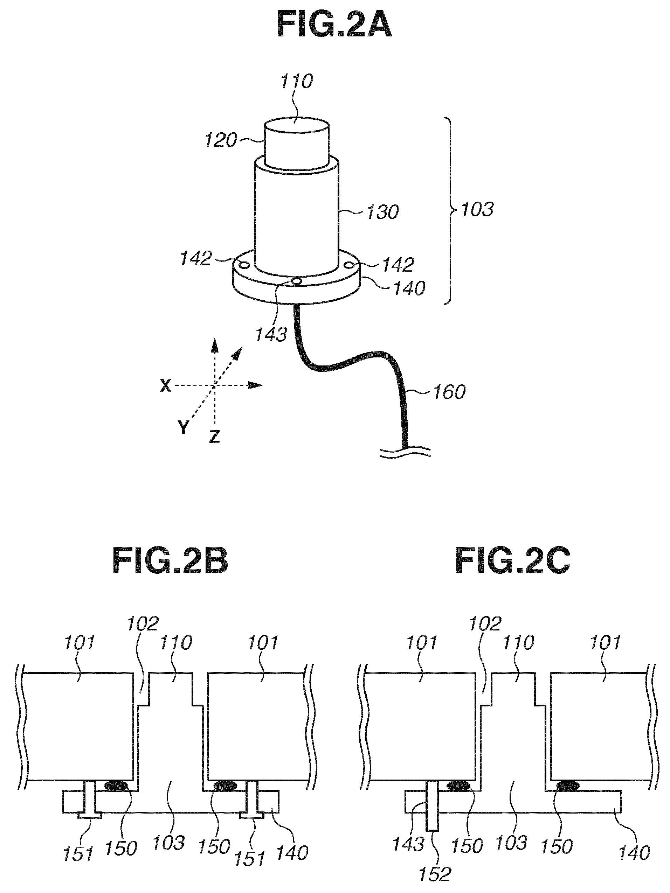

A second exemplary embodiment is different from the first exemplary embodiment in a structure that includes a positioning or alignment mechanism between a support member 101 and an acoustic wave transducer unit 103. As for others, the second embodiment is the same as the first embodiment. Fig. 2A is a schematic view illustrating the acoustic wave transducer unit 103 disposed in a photoacoustic wave probe 100 according to the present exemplary embodiment. In Fig. 2A, an aperture 143 corresponds to a positioning pin 152. Fig. 2A is a schematic view of the acoustic wave transducer unit 103. Figs. 2B and Fig. 2C are schematic cross sectional views taken along an X-Z plane and a Y-Z plane, respectively, and each illustrating the unit 103 shown in Fig. 2A mounted to the support member 101.

According to the present exemplary embodiment, there is provided an alignment mechanism between the support member 101 and the acoustic wave transducer unit 103. More specifically, the support member 101 has the positioning pin 152 corresponding to each unit 103, and a protruding plate portion 140 has the aperture 143 corresponding to the positioning pin 152. Accordingly, it is possible to position an ultrasonic transducer 110 at a desired location in the photoacoustic wave probe 100 even in a case where the ultrasonic transducer 110 is not arranged at a central part of the acoustic wave transducer unit 103. Therefore, there is no need to arrange the ultrasonic transducer 110 at a center part of the unit 103, so that restriction of the arrangement decreases and the size of the unit 103 can be reduced. Further, even in a case where the acoustic wave transducer unit 103 has a configuration in which a plurality of ultrasonic transducers 110 are arranged, there is no need to arrange the plurality of ultrasonic transducers 110 in a point-symmetric manner around a center of the unit 103. Therefore, restriction of arrangement of the unit 103 can be lowered, and the size of the unit 103 can be further reduced. Thus, according to the present embodiment, the alignment mechanism is a fitting mechanism including a set of pin and aperture, one being formed in one of a peripheral portion of the through hole in the support member and a flange portion of the acoustic wave transducer unit and the other being formed in the other thereof.

With the photoacoustic wave probe 100 according to the present exemplary embodiment, there can be provided a probe characterized in that deterioration of performance due to the assemblage is unlikely to occur and the ultrasonic transducer 110 can be disposed at a predetermined position. In addition, since restriction of arrangement of the ultrasonic transducer 110 in the acoustic wave transducer unit 103 can be reduced, the size of the unit 103 can be made small and the structure can be simplified.

According to the present exemplary embodiment, further remarkable effect can be obtained in a case where a capacitive transducer is employed as the ultrasonic transducer 110. The capacitive transducer is normally formed on a silicon chip, and the silicon chip is cut off by dicing in a rectangular outward form and used. Since a region is necessary on the chip for drawing the wiring outside from the capacitive transducer on the chip, a large restriction occurs where a rectangular chip with the capacitive transducer is arranged on a center part of the unit 103. In the photoacoustic wave probe 100 according to the present exemplary embodiment, restriction of arrangement of the ultrasonic transducer 110 in the unit 103 can be reduced, and hence, restriction of arrangement of the capacitive transducer can be significantly reduced.

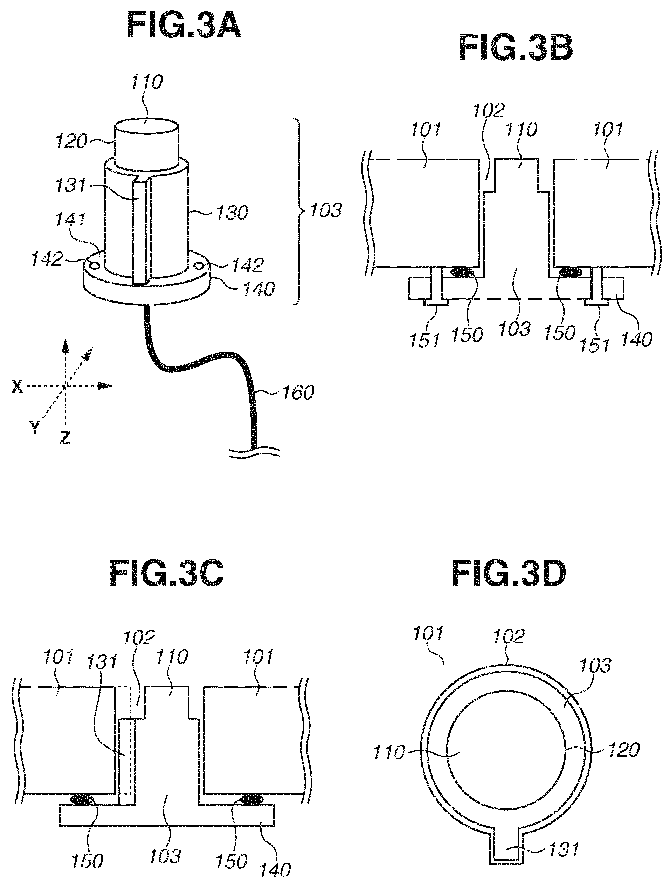

A third exemplary embodiment is different from the second exemplary embodiment in a structure of the alignment mechanism between a support member 101 and an acoustic wave transducer unit 103. As for others, the third embodiment is the same as the second embodiment. Fig. 3A is a schematic view illustrating the acoustic wave transducer unit 103 disposed in a photoacoustic wave probe according to the present exemplary embodiment. In Fig. 3A, a protrusion 131 extends in a longitudinal direction of a housing portion 130. Fig. 3A is a schematic view of the acoustic wave transducer unit 103. Figs. 3B and Fig. 3C are schematic cross sectional views taken along an X-Z plane and Y-Z plane, respectively, and each illustrating the unit 103 in Fig. 3A mounted to the support member 101. Further, Fig. 3D is a schematic diagram viewed in a radial direction from the center of the hemisphere of a photoacoustic wave probe 100, and illustrating the unit 103 in Fig. 3A mounted to the support member 101.

According to the present exemplary embodiment, side faces of the housing portion 130 and a through hole 102 respectively have concave and convex portions for alignment. The housing portion 130 of the unit 103 has a protrusion 131 for alignment. Meanwhile, inside the through hole 102 in the support member 101, a concave portion is formed at a position corresponding to the protrusion 131 of the unit 103. Therefore, advantageous effects can be obtained similarly to the second exemplary embodiment in which the positioning pin 152 is used. Further, according to the present exemplary embodiment, since the alignment mechanism is disposed on the side face of the housing portion 130, there is no need to provide a region for disposing a pin in the support member 101. Therefore, a region for fixing the acoustic wave transducer unit 103 can be further reduced and an interval between the units can be narrowed.

According to the present exemplary embodiment, since a plurality of the units 103 can be arranged with a narrowed interval, the ultrasonic transducer units 103 can be arranged with high density. In addition, deterioration of performance due to the assemblage is unlikely to occur, and an ultrasonic transducer 110 can be arranged at a predetermined position. In the present exemplary embodiment, the housing portion 130 has the protrusion 131 and the concave portion is formed outside the through hole 102 in the support member 101, but the structure is not limited thereto. A configuration in which the housing portion 130 has the concave portion and the protrusion is formed inside the through hole 102 in the support member 101 can be similarly used.

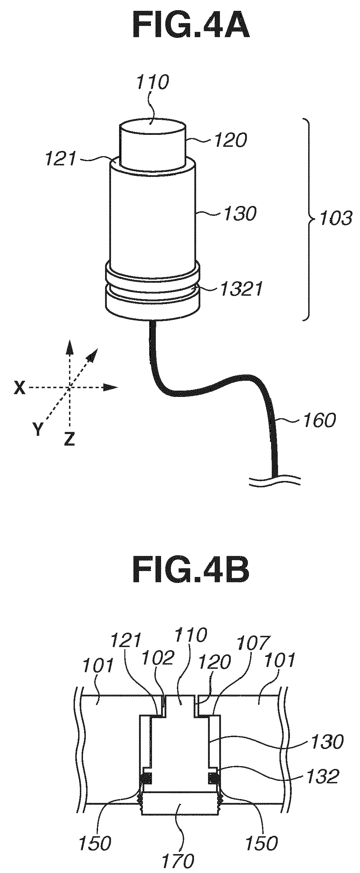

A fourth exemplary embodiment is different from the first embodiment in a shape of a through hole 102 in a support member 101. As for others, the present exemplary embodiment is the same as the first embodiment. Fig. 4A is a schematic view illustrating an acoustic wave transducer unit 103 according to the present exemplary embodiment. Fig. 4B is a schematic cross sectional view taken along an X-Z plane illustrating the unit 103 in Fig. 4A mounted to a support member 101. In Fig. 4A and Fig. 4B, there provided are a mount portion 132 for an O-ring 150 and a fixing component 170.

According to the present exemplary embodiment, a protruding plate portion 140 is not provided in the housing portion 130, and a step 107 is instead formed in a through hole 102 of the support member 101. A space inside the through hole 102 on the inner side of the support member 101 is smaller than a space on the outer side of the support member 101. An inside shape of the through hole 102 corresponds to an outward form of the acoustic wave transducer unit 103. The unit 103 has a plane 121 at a step formed between a leading edge portion 120 and a housing portion 130, and the plane 121 can impinge against the plane of the step 107 in the through hole 102 of the support member 101. Meanwhile, the outer end portion of the housing portion 130 in the unit 103 is pushed by a ring-shaped fixing component 170 such that the plane 121 at the step impinges against the support member 101. Under such a condition, a screw thread of the fixing component 170 is engaged with a bottom portion between screw threads formed in the through hole 102 of the support member 101, and hence, the unit 103 is fixed to the support member 101. Thus, the acoustic wave transducer unit 103 is held and fixed between the step 107 in the through hole 102 of the support member 101 and the fixing component 170.

According to the present exemplary embodiment, the mount portion 132 for mounting the O-ring 150 is formed on a side face of the housing portion 130 on the outer side of the support member 101. Between the mount portion 132 for the O-ring 150 and an inner face of the through hole 102 in the support member 101, the O-ring 150 is disposed. Accordingly, a gap between the support member 101 and the unit 103 is certainly sealed.

According to the present exemplary embodiment, the O-ring 150 is arranged on the side face of the acoustic wave transducer unit 103, so that a region for positioning the O-ring 150 can be reduced. Therefore, with the photoacoustic wave probe 100 according to the present exemplary embodiment, deterioration of performance of the ultrasonic transducer 110 is unlikely to occur at the time of assemblage of the support member 101 and the unit 103. Further, the ultrasonic transducers 110 can be arranged with high density.

A fifth exemplary embodiment is different from the fourth exemplary embodiment in a shape of a through hole 102 in a support member 101. As for the others, the present exemplary embodiment is the same as the fourth exemplary embodiment. Fig. 5A is a schematic view illustrating an acoustic wave transducer unit 103 of the present exemplary embodiment. Fig. 5B is a schematic cross sectional view illustrating the unit 103 fixed to the support member 101. Fig. 5C is a schematic cross sectional view taken along an X-Y plane at Z = Z1 in Fig. 5B. In Figs. 5A to 5C, a protrusion 133 is formed in a housing portion 130.

In the present exemplary embodiment, the protrusion 133 serving as a positioning unit is formed in the housing portion 130. The protrusion 133 is arranged on a side of a leading edge portion 120 which is on a side of a concave portion inner than a mount portion 132 for mounting an O-ring 150. Viewed from a side of an ultrasonic transducer 110, the protrusion 133 of the housing portion 130 does not project outwardly from a level of an outward form of the O-ring 150. Therefore, alignment can be achieved between the unit 103 and the support member 101, and the gap between the unit 103 and the support member 101 can be sealed with the O-ring 150.

With such a photoacoustic wave probe 100 according to the present exemplary embodiment, deterioration of performance of the ultrasonic transducer 110 is unlikely to occur at the time of assemblage of the support member 101 and the unit 103. In addition, the ultrasonic transducers 110 can be arranged at desired locations with high density.

According to the present exemplary embodiment, the housing portion 130 has the protrusion 133, and the support member 101 has the concave portion on the outer side of the through hole 102, but the structure is not limited thereto. Similar use can be made of a configuration in which the housing portion 130 has a concave portion, and the support member 101 has a protrusion on an inner side of the through hole 102.

Fig. 5D and Fig. 5E illustrate another example of the present exemplary embodiment. In this example, the mount portion 132 for mounting the O-ring 150 is arranged on a side closer to the leading edge portion 120 than the protrusion 133. Further, viewed from the side of the ultrasonic transducer 110, the protrusion 133 of the housing portion 130 is formed so as to project outwardly from a level of the outward form of the O-ring 150. Furthermore, the O-ring 150 can be disposed on a side closer to the leading edge portion 120, so that a region in which the unit 103 comes in contact with the acoustic matching material can be narrowed. Therefore, in the unit 103, a region necessary for waterproofing is only a vicinity of the leading edge portion 120, and the structure of the unit 103 can be simplified.

In the example described above, the housing portion 130 has the protrusion 133, and the support member 101 has the concave portion on the outer side of the through hole 102, but the structure is not limited thereto. Similar use can be made of a configuration in which the housing portion 130 has the concave portion, and the support member 101 has the protrusion on the inner side of the through hole 102. In this structure, viewed from a side of the ultrasonic transducer 110, the inside surface of the concave portion of the housing portion 130 is disposed so as to come in contact with an inner side of the outward form of the O-ring 150. Hence, the unit 103 can be aligned with the support member 101 and the gap between the unit 103 and the support member 101 can be sealed with the O-ring 150.

Fig. 5F and Fig. 5G illustrate yet another example of the present exemplary embodiment. Fig. 5F is a schematic view of the acoustic wave transducer unit 103. Fig. 5G is a diagram illustrating the unit 103 mounted to the support member 101, viewed in a radial direction from the center of the photoacoustic wave probe 100. This example is different from the above configuration in that the leading edge portion 120 is quadrangular. Further, in association with the shape of the leading edge portion 120, the shape of an opening in a step of the through hole 102 in the support member 101 is also quadrangular. In Fig, 5G, dotted lines represent the outward form of the housing portion 130 and the shape of the inside of the through hole 102 corresponding thereto, viewed from the side of the ultrasonic transducer 110. Thus, in the present exemplary example, the above alignment fitting mechanism is used between the outward form of the acoustic wave transducer unit 103 and a portion of the inside shape of the through hole 102 corresponding to the outward form of the unit 103.

In a case where the capacitive transducer formed on a quadrangular chip is used as the ultrasonic transducer 110, a useless space in the arrangement of the leading edge portion 120 can be reduced the most when the shape of the leading edge portion 120 is quadrangular. Similarly, the size of the acoustic wave transducer unit 103 can be reduced when the opening of the step in the through hole 102 is arranged so as to correspond to the quadrangular shape of the leading edge portion 120. However, in a case where the shape of the leading edge portion 120 and the opening of the step of the through hole 102 are quadrangular, a risk that the leading edge portion 120 comes in contact with the step portion of the through hole 102 increases if an insertion angle of the unit 103 shifts. In the present exemplary embodiment, the housing portion 130 is provided with the protrusion 133 for positioning of the unit 103 to the support member 101 for the time of when the protrusion 133 is inserted into the through hole 102. Therefore, insertion of the unit 103 can be attained without bringing the leading edge portion 120 into contact with the step portion. Further, according to the present exemplary embodiment, at the time of assemblage of the support member 101 and the unit 103, deterioration of performance of the ultrasonic transducer 110 is unlikely to occur. In addition, the ultrasonic transducers 110 can be arranged at predetermined positions with higher density.

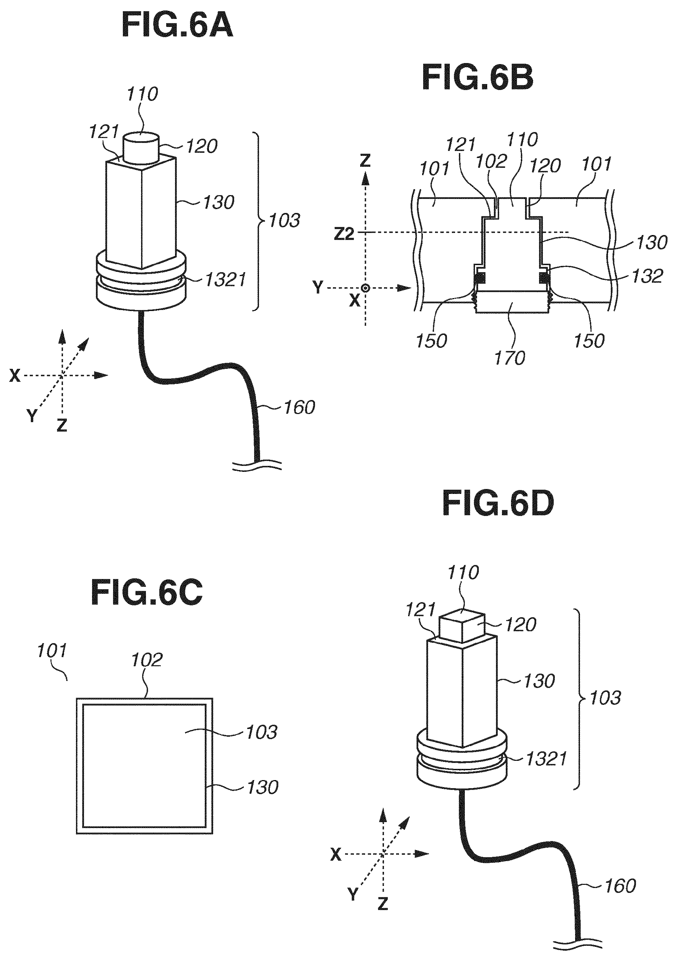

A sixth exemplary embodiment is different from the fifth embodiment in an alignment mechanism between a support member 101 and an acoustic wave transducer unit 103. As for the others, the present exemplary embodiment is the same as the fifth embodiment. Fig. 6A is a schematic view illustrating the unit 103 according to the present exemplary embodiment. Fig. 6B is a schematic cross sectional view illustrating the unit 103 fixed to the support member 101. Fig. 6B is a cross sectional view illustrating the unit 103, taken along a Y-Z plane in Fig. 6A. Fig. 6C is a schematic cross sectional view illustrating the unit 103, taken along an X-Y plane at Z = Z2 in Fig. 6B.

According to the present exemplary embodiment, an outward form of a housing portion 130 of the unit 103 is polygonal. An inside shape of a through hole 102 in the support member 101 is also polygonal corresponding to the outward form of the unit 103. Compared with the fifth exemplary embodiment, there is no need to form a concave or convex portion in an outward form of the housing portion 130 and the inside shape of the through hole 102. Hence, the structure can be simplified. Therefore, a region necessary for the alignment mechanism can be reduced and an interval between the units 103 can be narrowed. Thus, according to the present exemplary embodiment, the alignment fitting mechanism between polygon shapes is employed.

With such a photoacoustic wave probe 100 according to the present exemplary embodiment, at the time of assemblage of the support member 101 and the acoustic wave transducer unit 103, deterioration of performance of the ultrasonic transducer 110 is unlikely to occur. In addition, the ultrasonic transducers 110 can be arranged at desired locations with high density. Further, structures of the unit 103 and the through hole 102 in the support member 101 can be simplified, so that a probe with high reliability can be provided.

According to the present exemplary embodiment illustrated in Fig. 6A, the outward form of housing portion 130 and the inside shape of the through hole 102 are quadrangular, but the structure is not limited thereto. Similar effects can also be obtained as long as the polygon shape, such as triangle, pentagon, and hexagon, is used. Further, as illustrated in Fig. 6D, the outward form of the leading edge portion 120 of the unit 103 can be likewise formed polygonal, and the shape of the through hole 102 in the support member 101 can be formed polygonal corresponding to the form of the unit 103.

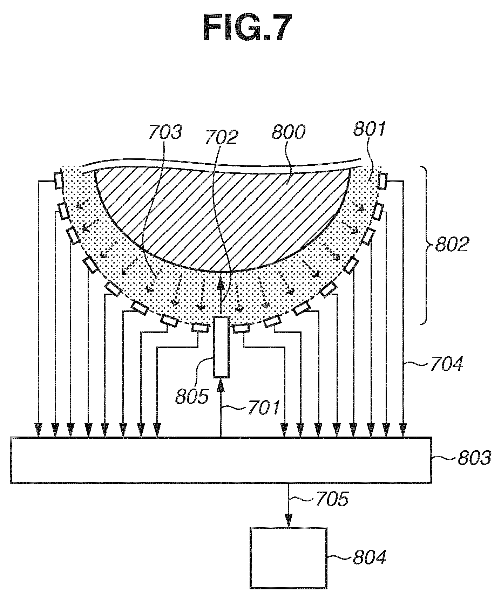

Any of the photoacoustic wave (ultrasonic) probes 100 described in the first to sixth exemplary embodiments can be used to receive a photoacoustic wave (ultrasound) due to the photoacoustic effect and applied to an object information acquisition apparatus. Referring to Fig. 7, an operation of an ultrasonic measurement apparatus according to the present exemplary embodiments will be specifically described. Based on a signal 701 of a light emission instruction, light 702 (pulse light) is emitted from a light source 805, and a measurement object 800 is irradiated with the light 702 through a medium 801. In the measurement object 800, a photoacoustic wave (ultrasound) 703 is generated due to irradiation with the light 702, and the ultrasound 703 is received by a plurality of capacitive transducers 802 in the ultrasonic probe. Information of the received wave, such as size, shape, and time, is transmitted as a reception signal 704 of the photoacoustic wave to an image information generating apparatus 803 that is a signal processing unit. Meanwhile, information (light emission information) of the light 702 generated in the light source 805, such as size, shape, time of the light, is stored in the an image information generating apparatus 803 for generating the image information of the photoacoustic signal. In the image information generating apparatus 803, an image signal of the measurement object 800 is generated based on the reception signal 704 of the photoacoustic wave and the light emission information, and reproduction image information 705 based on the photoacoustic signal is output. In an image display unit 804, an image of the measurement object 800 is displayed based on the reproduction image information 705 generated from the photoacoustic signal.

The photoacoustic wave (ultrasound) probes according to the above exemplary embodiments is characterized in that deterioration of characteristics of the ultrasonic transducer due to the installation of the ultrasonic transducer is unlikely to occur, whereby a photoacoustic wave can be accurately acquired. Therefore, an image with high quality can be obtained. In the above exemplary embodiments, a light irradiation portion for illuminating an object is arranged in the support member. Further, a photoacoustic wave generated in an object due to the photoacoustic effect is received by the acoustic wave probe according to the above exemplary embodiments to acquire information of the object.

As described above, any of the photoacoustic wave (ultrasonic) probes 100 according to the first to sixth exemplary embodiments can be used to receive a photoacoustic wave (ultrasound) due to the photoacoustic effect. In addition, ultrasound can be transmitted toward the object and ultrasound reflected by the object can be received. Such a configuration can be applied to an object information acquisition apparatus which acquires information of an object based on an acquired signal. The acoustic wave probe according to each of the exemplary embodiments performs both of reception of a photoacoustic wave generated in the object due to the photoacoustic effect and transmission/reception of ultrasound to/from the object. Thus, the acoustic wave probe acquires information of the object.

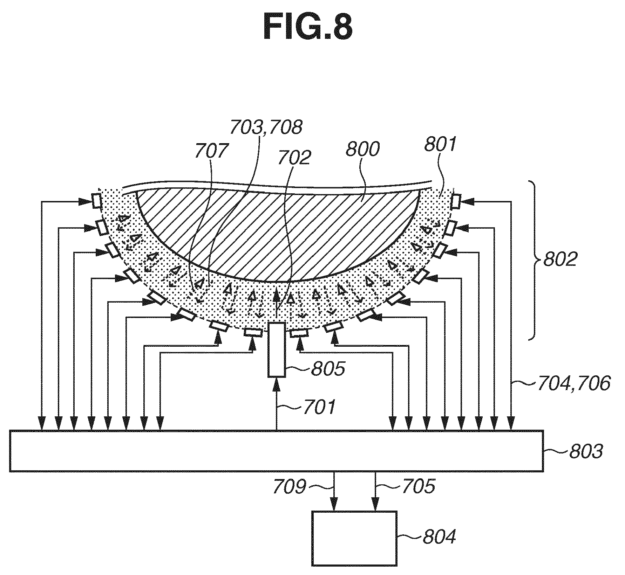

Fig. 8 is a schematic view illustrating the object information acquisition apparatus according to an eighth exemplary embodiment. In Fig. 8, there provided are a transmission and reception signal 706 of ultrasound, transmitted ultrasound 707, reflected ultrasound 708, reproduction image information 709 produced by transmission and reception of the ultrasound.

The object information acquisition apparatus of the present exemplary embodiment performs, in addition to reception of the photoacoustic wave, pulse echo (transmission and reception of ultrasound) to form an image. Reception of the photoacoustic wave in the present exemplary embodiment is the same as described in the seventh exemplary embodiment. Here, only pulse echo (transmission and reception of ultrasound) will be described. Based on the transmission signal 706 of ultrasound, the ultrasound 707 is transmitted from a plurality of capacitive transducers 802 toward a measurement object 800. In an inner portion of the measurement object 800, ultrasound is reflected due to a difference in intrinsic acoustic impedance of the substance present inside. The reflected ultrasound 708 is received by a plurality of the capacitive transducers 802, and information of the received signal, such as size, shape, time, is transmitted to the image information generating apparatus 803 as the reception signal 706 of the ultrasound. Meanwhile, information of the transmitted ultrasound, such as size, shape, and time, is stored in the image information generating apparatus 803 as the ultrasound transmission information. In the image information generating apparatus 803, an image signal of the measurement object 800 is generated based on the ultrasound reception signal 706 and the ultrasound transmission information, and the reproduction image information 709 generated by ultrasound transmission and reception is output.

In the image display unit 804, an image of the measurement object 800 is displayed based on two pieces of information of the reproduction image information 705 generated from the photoacoustic signal and reproduction image information 709 generated by transmission and reception of ultrasound. The photoacoustic wave (ultrasound) probe 100 according to the present exemplary embodiments is characterized in that deterioration of characteristics of the ultrasonic transducer due to the installation of the ultrasonic transducer is hard to occur, so that a photoacoustic wave can be accurately acquired. Further, ultrasound can be accurately transmitted and received using the same probe. Therefore, a photoacoustic image and an ultrasonic image having the same coordinate system can be generated with high quality.

According to the present exemplary embodiment, the capacitive transducer 802 can be also configured such that at least ultrasound from the object is received, and the processing unit can be also configured such that information of the object is acquired using an ultrasound reception signal from the capacitive transducer 802. In this case, the capacitive transducer 802 can emit ultrasound toward an object, but it is also possible to cause another transducer to emit ultrasound. Further, it is also possible for the capacitive transducer 802 to perform ultrasound reception without performing reception of a photoacoustic wave. As described above, the acoustic wave probe detects a photoacoustic wave and/or ultrasound from an object located at a position facing the concave side of the support member with the hemispherical shape or the like. Further, the signal processing unit produces an image of the object, such as a living tissue, from signals of a photoacoustic wave and/or ultrasound detected by the probe.

Hereinafter, exemplary embodiments different from the above exemplary embodiments will be described.

These exemplary embodiments will be described referring to the drawings. A photoacoustic wave probe according to the present exemplary embodiments has a plurality of transducer units including a transducer which receives a photoacoustic wave generated due to irradiation of an object with light from a light source and outputs an electric signal. A support member for supporting the plurality of transducer units is configured such that directional axes of the plurality of transducer units gather together. The plurality of transducer unit is arranged in inner portions of apertures formed in the support member and is disposed such that the plurality of transducer units does not protrude from the support member toward a side on which directional axes gather together. Since the plurality of transducer units does not protrude inwardly from the support member, it is possible to suppress turbulent flow of an acoustic matching material disposed in a space between the support member and the object, and unevenness of temperature distribution of the acoustic matching material. Specific configurations will be described.

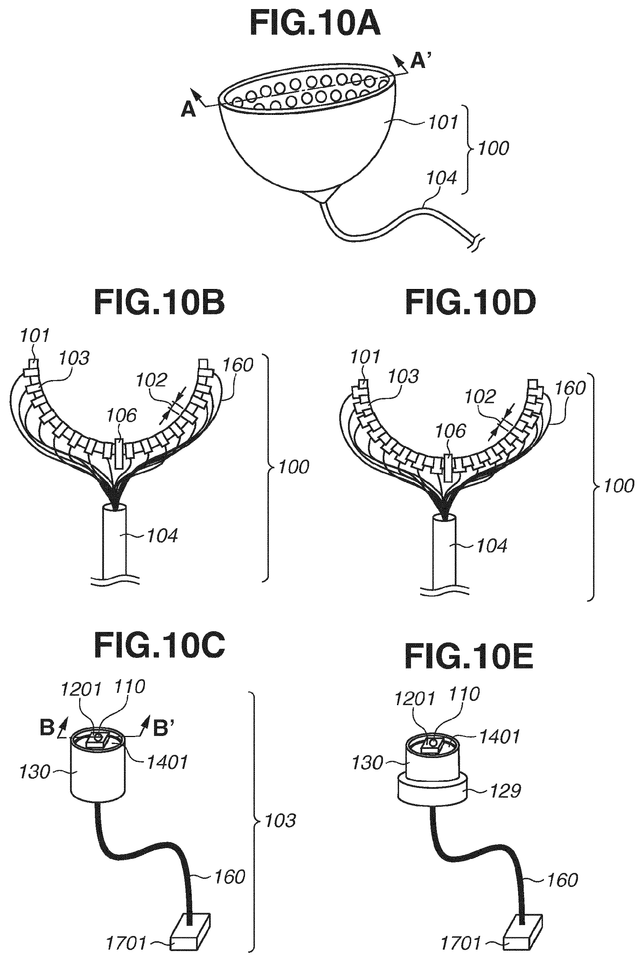

A photoacoustic wave probe 100 according to a ninth exemplary embodiment will be described with reference to Figs. 10A to 10E. Fig. 10A is a schematic view illustrating an outward form of the photoacoustic wave probe 100 according to the present exemplary embodiment. Fig. 10B is a schematic cross sectional view taken along an A-A' cross section illustrating the photoacoustic wave probe 100 in Fig. 10A. Fig. 10C is a schematic view illustrating a transducer unit 103. Further, Fig. 10D is a diagram illustrating the photoacoustic wave probe 100 having another example structure according to the present exemplary embodiment. Fig. 10E is a schematic view illustrating the transducer unit 103 having yet another example structure. In Fig. 10A, a cable and so forth are omitted.

In Figs. 10A to 10E, the photoacoustic wave probe 100 includes a support member 101 with a hemispherical shape, an aperture (through hole) 102, the transducer unit 103, a cable 160, a cable bundle 104, a connector 1701, a light source 106, and a transducer 110.

The hemispherical support member 101 includes a plurality of through holes 102 for positioning the transducers 110 in predetermined positions.

An outward form of the transducer unit 103 corresponds to the shape of the through hole 102, and each transducer unit 103 is inserted and fixed in a different one of the through holes 102. The transducer unit 103 includes at least a transducer 110, and the transducer unit 103 is arranged such that a directional axis of the transducer 110 passes through a vicinity of the center of the hemisphere of the support member 101.

The transducer unit 103 has a housing portion 130 with a columnar shape, and a chip and a CMUT formed on the chip can be disposed on a bottom face of the housing portion 130. Further, as described below, it is also possible to arrange the CMUT on an interposer and arrange a detecting circuit of the CMUT on a rear surface of the interposer opposite to a surface on which the CMUT is arranged.

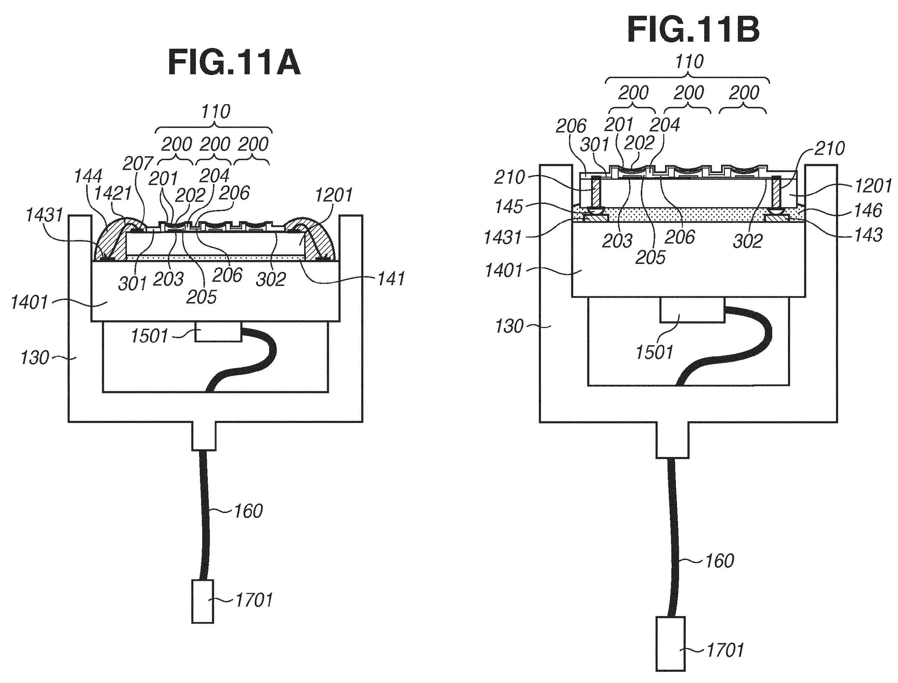

Further, an O-ring can be disposed on a side face of the housing portion 130 such that inflow of acoustic matching material such as water can be suppressed, and the housing portion 130 can be fixed to the support member 101. Furthermore, as illustrated in Fig. 11A, an area of a main surface of the interposer can be made larger than an area of a main surface of the chip.

As illustrated in Fig. 10C, the housing portion 130 with a columnar shape can be replaced by a housing portion with a cylindrical shape. A configuration with at least two connected cylindrical portions of different diameters as illustrated in Fig. 10E can also be used. Further, the shape of the housing portion 130 can be prismatic. Furthermore, a side face of the housing portion 130 with a cylindrical shape can have a protrusion.

According to the present exemplary embodiment, a cross sectional shape of the through hole 102 is a perfect circle. The transducer unit 103 has the cylindrical housing portion 130, and the transducer 110 is arranged on a center part of a face of the cylindrical housing portion 130.

The light source 106 is arranged in a center part (vertex) of the hemispherical support member 101. The light source 106 can be any of those that can emit light, such as a solid laser, a gas laser, a semiconductor laser, and an LED. Examples of the light source according to the present exemplary embodiment include a light source having a configuration in which light from a light emitting portion arranged outside is guided using an optical fiber or a multi-articulated arm with mirrors at its joints. According to the present exemplary embodiment, the light source 106 is disposed at the center part (vertex) of the hemispherical support member 101, but the arrangement is not limited thereto. For example, a single light source 106 or a plurality of light sources 106 can be arranged at any region of the hemispherical support member 101 where no transducer unit 103 is disposed. Further, though the light source 106 illustrated in Figs. 10B and 10D projects toward a side of the object, it is possible to adopt a configuration in which the light source 106 does not project toward the side of the object from the level of the support member 101. Further, the light source 106 can be disposed at a position other than the center part (vertex) of the hemispherical support member 101.

According to the present exemplary embodiment, the transducer unit 103 which is separately provided from the support member 101 is mounted in the through hole 102 formed in the support member 101. Therefore, the support member 101 can have a very simple structure having apertures formed in a hemispherical member. Further, since the support member 101 and the transducer unit 103 are separate from each other, an operation-tested transducer unit 103 can be selected. Hence, the yield of the photoacoustic wave probe 100 can be easily improved. Further, in a case where the unit 103 is broken down, replacement can be easily executed. Moreover, a photoacoustic wave probe 100 with a different sensor interval can be easily prepared by changing only positions of the through holes 102 in the support member 101. Similarly, since a hemispherical probe with a different radius can be provided by preparing only the support member 101 with a different radius, the same transducer unit 103 can be used irrespective of the configuration of the support member 101.

(Shape of Support Member)

A shape of the support member 101 will be described. According to the present exemplary embodiment, the shape of the support member 101 is hemispherical, but not limited thereto as long as directional axes of transducers 110 are arranged to gather together. The shape can be a truncated cone shape, a prismoid shape, or a semicircular arching shape. Further, in the hemispherical shape of the support member 101, an angle formed between a line connecting a center of a sphere of the hemisphere shape and a vertex of the sphere and a line connecting center of the sphere and an edge of the hemisphere can be 90 degrees, but not limited thereto. The angle can also be less than 90 degrees or larger than 90 degrees. In this specification, the spherical shape is not limited to a perfect sphere. It is possible to adopt an ellipsoid, or a shape having a concave and a convex surface on an approximately spherical surface as long as the shape can be considered to be equivalent to a sphere. According to the present exemplary embodiment and exemplary embodiments described below, the support member 101 has a hemispherical shape.

(Shape of Aperture)

According to the present exemplary embodiment, the aperture 102 in the support member 101 is a through hole, but the aperture 102 is not limited to a completely-penetrating aperture. Further, the cross sectional shape of the through hole (aperture) 102 is described as a perfect circle in the above description, but the shape is not limited thereto. The cross sectional shape can be ellipse, or rectangle (such as triangle, quadrangle, polygon, and trapezoid and the like).

(Transducer)

A transducer will be described. The transducer 110 according to the present exemplary embodiment can be any ultrasonic transducer as long as it can receive or transmit ultrasound. For example, a PZT type ultrasonic probe, or a PVDF type ultrasonic probe can be used. Particularly, a CMUT is preferable. According to the present exemplary embodiments described below, the transducer 110 is a CMUT.

In Fig. 10C, the transducer unit 103 includes a chip 1201, a housing portion 130, an interposer 1401, a cable 160, and a connector 1701. Further, as described below, the chip 1201 and the interposer 1401 are bonded to each other with an adhesive.

The transducer unit 103 includes the housing portion 130 with a cylindrical shape, and the chip 1201 is disposed inside the housing portion 130. The CMUT 110 is formed on the chip 1201. On a bottom face of the housing portion 130 with the cylindrical shape, the chip 1201 is disposed such that the CMUT 110 faces outward. The housing portion 130 does not cover a surface of the CMUT 110 so that a photoacoustic wave from outside of the transducer unit 103 reaches a surface of the CMUT 110. Further, the cable 160 for connection to the outside extends out from the other bottom face of the housing portion 130.

A shape of the housing portion 130 will be described. In Fig. 10C, the shape of the transducer unit 103 is cylindrical, but not limited thereto. For example, as shown in Fig. 10E, the diameter of a portion on a side from which the cable 160 extends out can be made larger than the diameter of the through hole 102. Accordingly, using a simple structure, the transducer unit 103 can be accurately aligned with the support member 101. Thus, with , with a simple structure, a plurality of transducers 110 can be accurately arranged over the hemispherical photoacoustic wave probe 100 and information from the object can be accurately acquired.

(CMUT)

The CMUT 110 will be described. The CMUT 110 is fabricated on a silicon chip using a Micro Electro Mechanical Systems (MEMS) process that is an application of a semiconductor process. Figs. 11A and 11B illustrate the CMUT 110.

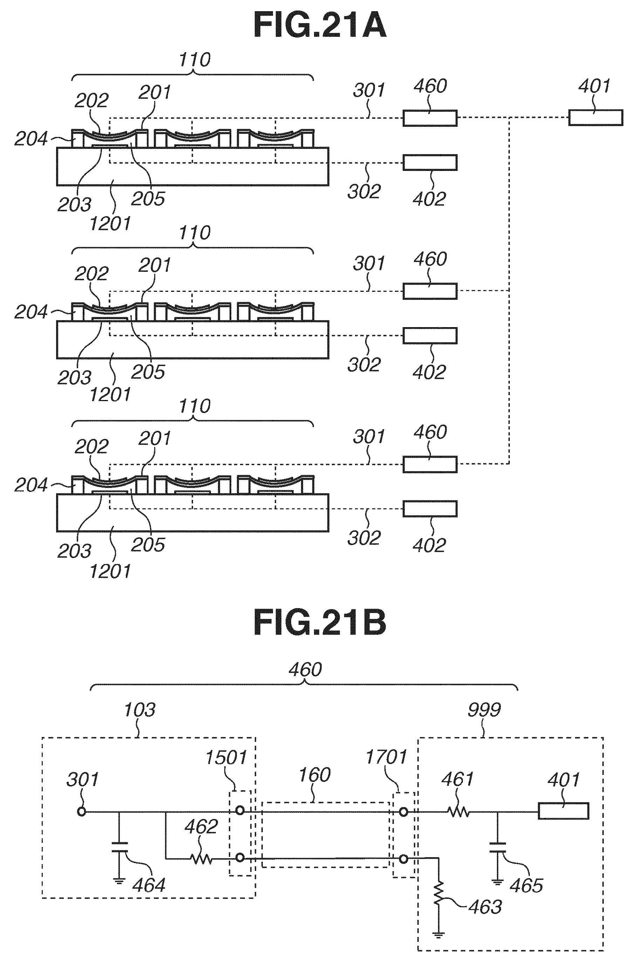

Fig. 11A is a schematic view illustrating the CMUT 110. In Fig. 11A, the CMUT 110 includes a chip (substrate) 1201, a vibration film 201, a first electrode (upper electrode) 202, a second electrode (lower electrode) 203, and a support portion 204 for supporting the vibration film 201. Further, the CMUT 110 includes a gap 205, a first wiring 301 connected to the first electrode 202, and a second wiring 302 connected to the second electrode 203. The first wiring 301 and the second wiring 302 are respectively connected to a direct current (DC) voltage generating unit 401 (illustrated in Fig. 21) and a detecting circuit 402 (which is a current-voltage converting circuit illustrated in Fig. 21).

The vibration film 201 is supported by the support portion 204 on the chip 1201 and vibrates when it receives ultrasound. The first electrode 202 is disposed on the vibration film 201, and the second electrode 203 is disposed on the chip 1201 so as to face the first electrode 202. A set composed of the first electrode 202 and the second electrode 203 facing each other with the vibration film 201 and the gap 205 interposed between them is referred to as a cell 200. In Fig. 11A, the CMUT 110 includes three cells 200, but the number of cells can be one, two, or more than three. The CMUT 110 including a plurality of cells may be referred to as a CMUT array. Further, a cell group including a plurality of cells may be referred to as an element.

The first electrode 202 is drawn outside the chip 1201 through the first wiring 301 and connected to the DC voltage generating unit 401 (illustrated in Fig. 21). The second electrode 203 is drawn outside the chip 1201 through the second wiring 302 and connected to the detecting circuit 402 (illustrated in Fig. 21). Using the DC voltage generating unit 401, electric potential difference of a voltage between several tens voltages and several hundred voltages is generated between the first electrode 202 and the second electrode 203. When the vibration film 201 and the first electrode 202 are vibrated, a distance between the first electrode 202 and the second electrode 203 changes, and electrostatic capacitance between the electrodes changes. Since the electric potential difference is present between the electrodes, a small current is generated corresponding to a change in capacitance. The small current is converted to voltage by the detecting circuit 402 to which the second electrode 203 is connected, and the voltage is output.

The CMUT 110 used in the present exemplary embodiments has characteristics that responsiveness at the time of reception of ultrasound is preferable and a reception frequency band is broad, compared with a piezoelectric ultrasonic transducer which is widely used at present.

The chip 1201 is fixed to the interposer 1401 with an adhesive 141. An electrode of the CMUT 110 on the chip is electrically connected to the wiring on the interposer 1401 by a wire 1421. The wire 1421 can be a gold wire or the like and easily disposed using wire bonding technology. According to the present exemplary embodiments, the interposer 1401 is a multilayer circuit substrate in which a glass epoxy-based resin and a conductive layer of a copper film are layered. The wiring connected to the CMUT 110 on the surface of the interposer 1401 is electrically connected to a connector 1501 on a bottom surface of the interposer 1401, through wiring (not shown) in the interposer 1401. According to the present exemplary embodiments, the wiring from the chip 1201 is connected to the cable 160 by the connector 1501 arranged on the bottom surface of the interposer 1401, via the interposer 1401.

Accordingly, in the present exemplary embodiment, since the wiring disposed in the interposer 1401 is used, a minute electrode of the CMUT 110 on the chip 1201 can be connected to a thick cable for connection to an external apparatus (not shown) of the photoacoustic apparatus, with a simple structure, and can be drawn outside.

As described above, according to the present exemplary embodiment, the hemispherical support member 101 includes a plurality of through holes 102 and the transducer unit is inserted and fixed in the through hole 102. Therefore, there can be provided the photoacoustic wave probe 100 in which the transducer 110 can be arranged at a predetermined position using a simple structure.

Another example of the CMUT 110 will be described with reference to Fig. 11B. In this configuration, the chip 1201 has penetrating wiring 210, and the first wiring 301 and the second wiring 302 connected to the electrodes of CMUT 110 on the chip 1201 can be drawn out to a bottom surface of the chip 1201. The bottom surface of the chip 1201 is electrically connected to the electrode 1431 on the interposer 1401 by a solder bump 145. A space between the bottom surface of the chip 1201 and the interposer 1401 is preferably filled with insulating underfill material 146. The underfill material 146 can enhance insulation property between the electric connection portion and outside whereby such a possibility can be reduced that the electric connection portion is badly influenced by humidity and connection failure occurs.

According to the above another example of the present exemplary embodiments, there is no projection on a side of the surface of the CMUT 110, viewed from a side of the object. Hence, there can be provided a photoacoustic wave probe characterized in that deterioration of receiving characteristics of a photoacoustic wave due to a projection does not occur.

In Fig. 11B, the solder bump 145 is used, but connection is not limited thereto. A gold bump, an electroconductive paste, or the like can be used. In a case where contamination due to flux at the time of using solder and processing temperature of reflow become problems, the electric connection portion can be configured by applying the above configuration in which solder is not used.

A tenth exemplary embodiment relates to a circuit disposed in the interposer 1401.

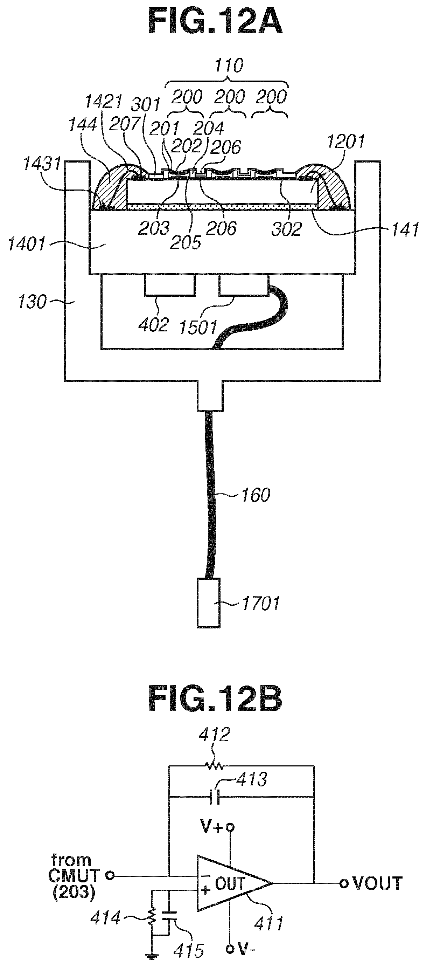

Fig. 12A is a schematic cross sectional view illustrating a photoacoustic wave probe 100 according to the present exemplary embodiment.

According to the present exemplary embodiment, as described in Fig. 12A, a detecting circuit 402 connected to a CMUT 110 is arranged on a surface of an interposer 1401 opposite to a surface on which the CMUT 110 is arranged. The CMUT 110 is characterized in that a receptible frequency band is broad, compared with a piezoelectric ultrasonic transducer which is widely used at present. However, reception performance of the CMUT 110 can be easily deteriorated by parasitic capacitance of the second wiring 302 which electrically connects the CMUT 110 to the detecting circuit 402. According to the present exemplary embodiment, since the chip 1201 and the detecting circuit 402 are arranged with the interposer 1401 provided between them, the CMUT 110 and the detecting circuit 402 can be disposed close to each other at a minimum distance.

In the photoacoustic wave probe according to the present exemplary embodiment, the detecting circuit 402 is disposed close to the CMUT 110 and integrally arranged with the CMUT 110. Accordingly, the CMUT 110 with an excellent reception characteristics in a broad band region can be provided.

The detecting circuit according to the present exemplary embodiment illustrated in Fig. 12A will be described with reference to Fig. 12B. In Fig. 12B, the detecting circuit 402 is a transimpedance circuit using an operation amplifier 411. In FIG.12B, the detecting circuit 402 includes a resistor 414 and a capacitor 415.

As illustrated in Fig. 12B, the detecting circuit 402 can be formed with the transimpedance circuit using the operation amplifier 411. In the transimpedance circuit using the operation amplifier 411, a resistor 412 and a capacitor 413 are disposed in a parallel form in a negative feedback portion of the operation amplifier 411. Current input through the feedback portion is converted to voltage. Due to feedback characteristics of the operation amplifier 411, influence of parasitic capacitance in the input wiring on the current-voltage conversion efficiency can be reduced by using a broad-band operation amplifier. Therefore, as to reception characteristics of the CMUT 110 with a broad frequency width, it is possible to obtain excellent reception characteristics in which deterioration of receiving sensitivity is little. According to the present exemplary embodiment, since the transimpedance circuit with the operation amplifier 411 is used in the detecting circuit 402, influence of parasitic capacitance in the input terminal of the detecting circuit 402 is little. Therefore, the photoacoustic probe characterized in that little deterioration of reception characteristics due to parasitic capacitance can be provided.

In the transimpedance circuit using the operation amplifier 411, influence of parasitic capacitance in the second wiring 302 on current-voltage conversion characteristics can be reduced by using a high-speed operation amplifier. Therefore, CMUT 110 can have excellent reception characteristics.

According to the present exemplary embodiment, the CMUT 110 with more excellent reception characteristics can be provided.

An eleventh exemplary embodiment relates to a circuit which is provided in an interposer 1401. As for others, this embodiment is the same as the tenth embodiment, and accordingly description thereof is omitted.

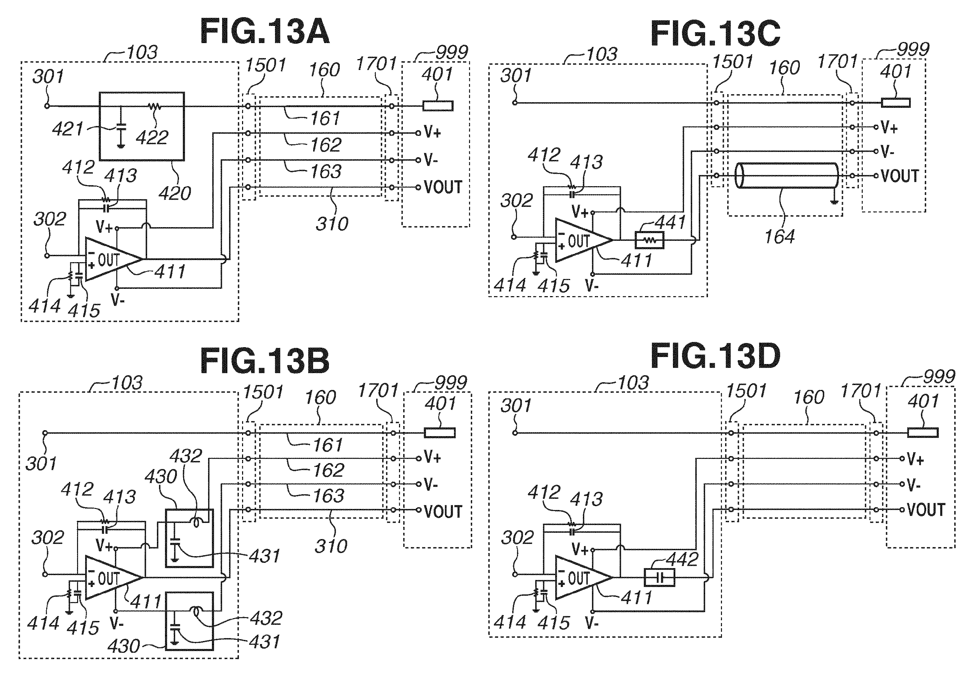

Fig. 13A to 13C are circuit diagrams each illustrating a circuit in a photoacoustic wave probe 100 according to the present exemplary embodiment.

According to the present exemplary embodiment, as illustrated in Fig. 13A, a circuit 420 for stabilizing DC voltage is disposed on the interposer 1401.

A cable 160 according to the present exemplary embodiment contains a set of a DC voltage applying line 161, power supply lines 162 and 163 for a current-voltage converting circuit, and a signal line 310 for output.

Since there is a long distance from a transducer unit 103 arranged in a dispersed manner on a hemispherical support member 101 to a connector 1701 connected to the external apparatus (not shown), a certain amount of the length of a cable 160 connecting the transducer unit 103 and the connector 1701 is required. Therefore, due to wiring resistance of the wires in the cable 160, DC voltage Vb1 on a side of the interposer 1401 becomes different from DC voltage Vb0 at the connector 1701 on a side of the apparatus. Reception characteristics of a CMUT 110 is largely influenced by electric potential difference applied between a first electrode 202 and a second electrode 203. Therefore, if DC voltage Vb1 applied to the CMUT 110 differs from DC voltage Vb0 generated by a DC voltage generating unit 401, desired reception characteristics cannot be obtained. According to the present exemplary embodiment, since a circuit 420 for stabilizing DC voltage is disposed, the DC voltage Vb1 applied to the CMUT 110 can be caused to coincide with the DC voltage Vb0 generated by the DC voltage generating unit 401. Accordingly, influence of wiring resistance in the cable 160 on reception characteristics of the CMUT 110 can be reduced.

Input impedance of the circuit 420 for stabilizing DC voltage according to the present exemplary embodiment only needs to be set sufficiently smaller than wiring resistance of the cable 160. More specifically, as illustrated in Fig. 13A, a high withstand voltage capacitor 421 and a resistor 422 can readily form the input impedance. According to the present exemplary embodiment, the configuration is not limited to that illustrated in Fig. 13A. Another circuit can be similarly used as long as DC voltage Vb1 on the side of the interposer 1401 can be made close to DC voltage Vb0 in the connector on the side of the apparatus.

According to the present exemplary embodiment, it is possible to provide the photoacoustic wave probe characterized in that deterioration of reception characteristics of the CMUT 110 is reduced.

Another example of the present exemplary embodiment will be described with reference to Fig. 13B. In Fig. 13B, a stabilizing circuit 430 for stabilizing detecting circuit power supply (operation amplifier) is disposed on the interposer 1401. Current for driving the detecting circuit 402 needs to be stably supplied to the power supply for the detecting circuit 402. If wiring resistance appears in the cable 160, current supplied to the detecting circuit 402 becomes unstable and current-voltage conversion characteristics of the detecting circuit 402 is likely to be influenced. According to the present exemplary embodiment, since the stabilizing circuit 430 for the detecting circuit power supply is disposed, necessary current can be stably supplied to the detecting circuit 402. Thus, desired current-voltage conversion characteristics can be obtained in the detecting circuit 402. As illustrated in Fig. 13B, the stabilizing circuit 430 for the detecting circuit power supply according to the present exemplary embodiment can be easily configured using a capacitor 431 and a coil 432. The circuit according to the present exemplary embodiment is not limited to the circuit illustrated in Fig. 13B. A circuit having the same function as above can be used.

According to another example of the present exemplary embodiment, current output from the CMUT 110 can be efficiently converted to voltage by the detecting circuit 402, whereby excellent reception characteristics can be obtained.

Yet another examples of the present exemplary embodiment will be described referring to Figs. 13C and 13D. In Fig. 13C, an impedance matching resistor 441 for impedance matching to a signal line 310 is connected between an output terminal of the detecting circuit 402 and the signal line 310. It is preferable that a coaxial cable 164 is used as the signal line 310 for supplying an output signal of the detecting circuit 402 to the side of the apparatus. When the coaxial cable 164 is used, noise from outside can be prevented from being superimposed on the signal line 310. On the other hand, if the wiring becomes long, it is a little likely that reflection in the wiring occurs and signal characteristics is deteriorated. In Fig. 13C, there is provided the impedance matching resistor 441 matched to the wiring impedance of the signal line 310 that is a coaxial cable. Thus, since the output terminal of the detecting circuit 402 is provided with the impedance matching circuit, deterioration of the output signal due to mismatching between the interposer 1401 and the signal line 310 can be reduced. Accordingly, it is possible to provide an ultrasonic transducer unit 103 characterized in that deterioration of a detection signal in the cable 160 is reduced.

Further, as illustrated in Fig. 13D, it is possible to adopt another configuration in which the output terminal of the detecting circuit 402 includes an AC coupling circuit. More specifically, in this configuration, a capacitor 442 is connected between the output terminal of the detecting circuit 402 and the signal line 310. To receive a photoacoustic wave, the CMUT 110 only needs to detect a signal in a certain frequency range or more, e.g., from 10 kHz or more to 100 MHz or less, or from 100 kHz or more to 20 MHz or less. In Fig. 13D, since the capacitor 442 is disposed at the output terminal of the detecting circuit 402, a DC component of the output voltage can be eliminated. If a DC component is present in the output voltage, current flows in the wiring at all times and power consumption increases. In contrast, since a DC component of the output voltage is eliminated in the circuit structure of Fig. 13D, it is possible to eliminate current flowing in the wiring at all times and thereby reduce the power consumption. Therefore, the ultrasonic transducer unit 103 with small power consumption can be provided.

A twelfth exemplary embodiment relates to a member disposed on a surface of a CMUT 110. As for others, the present exemplary embodiment is the same as any of the ninth to eleventh exemplary embodiments.

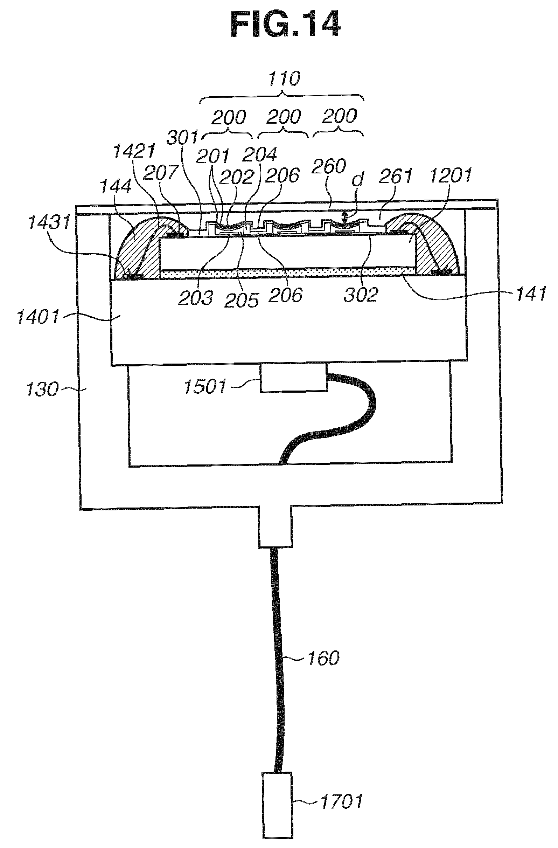

Fig. 14 is a schematic view illustrating an acoustic wave transducer unit 103 disposed in a photoacoustic wave probe 100 according to the present exemplary embodiment. In Fig. 14, the acoustic wave transducer unit 103 includes an insulating film 260 and a silicone rubber layer 261.

According to the present exemplary embodiment, the insulating film 260 is disposed on a transducer 110 with the silicone rubber layer 261 inbetween them.

The insulating film 260 can be a thin insulating film. Material, such as polyethyleneterephthalate (PET), polyimide (PI), polyethylene (PE), polymethylpentene (TPX) or the like, which can be thinned, can be used for the insulating film 260. The thickness of the insulating film 260 only needs to be sufficient to the wavelength of ultrasound to be used and is preferably from several micrometers to ten and several micrometers.

The silicone rubber layer 261 is excellent in transmission characteristics for a photoacoustic wave and firmly bonds the insulating film 260 to the CMUT 110. According to the present exemplary embodiment, a vibration film 201 on a side of the CMUT 110 bonded to the insulating film 260 is thin and is largely influenced by a member disposed on the vibration film 201. Therefore, in a case where an adhesive, such as an epoxy-based adhesive or the like, whose hardness after cured is high is used, vibration characteristics of the vibration film 201 is influenced, and, as a result, reception sensitivity may be significantly lowered. Since hardness of a silicone rubber after cured is low, in a case where a silicone rubber is disposed on the surface of the CMUT 110, vibration characteristics of the vibration film 201 in the CMUT 110 is unlikely to be influenced. Further, in a case where the thickness of the silicone rubber layer 261 is forty (40) micrometers or less, influence on transmission characteristics of ultrasound is small. Such thickness is therefore more preferable. Further, in a case where an interval between the chip 1201 and the insulating film 260 is too small, reception characteristics of the vibration film 201 for photoacoustic wave (ultrasound) is likely to be influenced. Hence, it is preferable that the thickness of the silicone rubber layer 261 is twenty (20) micrometers or more. From the above reasons, it is particularly preferable that the thickness of the silicone rubber layer 261 according to the present exemplary embodiment is from about twenty micrometers to about forty micrometers under the consideration that the silicone rubber layer 261 is used in the photoacoustic wave probe.

According to the present exemplary embodiment, since the insulating film 260 is disposed, it is possible to electrically insulate outside from the surface of the CMUT 110 having an electrode to which high voltage is applied. Therefore, it is possible to provide the photoacoustic wave probe characterized in that safety to the object is highly assured.

Further, according to the present exemplary embodiment, the silicone rubber layer 261 is arranged between the insulating film 260 and the chip 1201. Therefore, it is possible to provide the photoacoustic wave probe 100 characterized in that reception characteristics for a photoacoustic wave (ultrasound) is little deteriorated and, at the same time, adhesive property of the insulating film 260 to the chip 1201 is ensured.

A thirteenth exemplary embodiment relates to a mount portion for mounting a transducer unit 103 to a support member 101 illustrated in Fig. 10A. As for others, the present exemplary embodiment is the same as any of the ninth to twelfth embodiments.

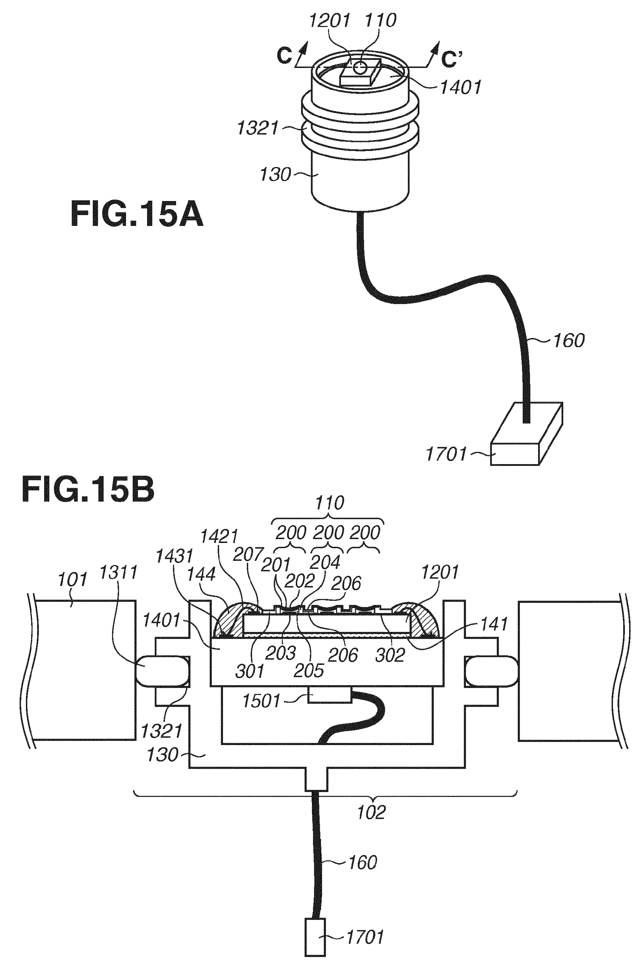

Fig. 15A is a perspective view illustrating an ultrasonic transducer 110 of the present exemplary embodiment. Fig. 15B is a cross sectional view taken along a line C-C' in Fig. 15A illustrating the support member 101 and an O-ring 1311 disposed between the transducer 110 and the support member 101.

According to the present exemplary embodiment, the O-ring 1311 is arranged between the transducer unit 103 and a through hole 102 in the support member 101. A housing portion 130 of the transducer unit 103 has a groove 1321 for arranging the O-ring 1311. The transducer unit 103 with the O-ring 1311 disposed in the groove 1321 is fixed to the support member 101 in a manner such that the transducer unit 103 is pressed by a pressing ring(not shown) from an outside (a side opposite to the side of the object) of the hemispherical support member 101 to the side of the support member 101. A gap between the through hole 102 and the housing portion 130 of the transducer unit 103 is set narrower than the thickness of the O-ring 1311. Therefore, the O-ring 1311 is slightly crushed by an inside surface of the through hole 102 and an outside surface of the housing portion 130, and the gap between the inside surface of the through hole 102 and the outside surface of the housing portion 130 is completely sealed by the O-ring 1311.

The photoacoustic wave probe 100 is used in such a manner that a space between the hemispherical support member 101 and the object is filled with the ultrasound gel having a high ultrasound transmission property so that a photoacoustic wave is not attenuated. According to the present exemplary embodiment, no flow of liquid occurs to a side of the support member 101 opposite to the object side because of the presence of the O-ring 1311. Hence, leakage of the ultrasound gel toward the side opposite to the object side is prevented. Therefore, it is possible to prevent adhesion of the ultrasound gel on electric components and mechanical components in the apparatus disposed outside the support member 101, whereby a change in characteristics and breakdown can be reduced. Further, since leakage of the ultrasound gel is suppressed, the space between the hemispherical support member 101 and the object can be certainly filled with the ultrasound gel. Hence, the photoacoustic wave probe 100 capable of reliably detecting a photoacoustic wave can be provided.

A fourteenth exemplary embodiment relates to a member in a transducer unit 103. As for others, the present exemplary embodiment is the same as any of ninth to thirteenth exemplary embodiments, and accordingly description thereof is omitted.

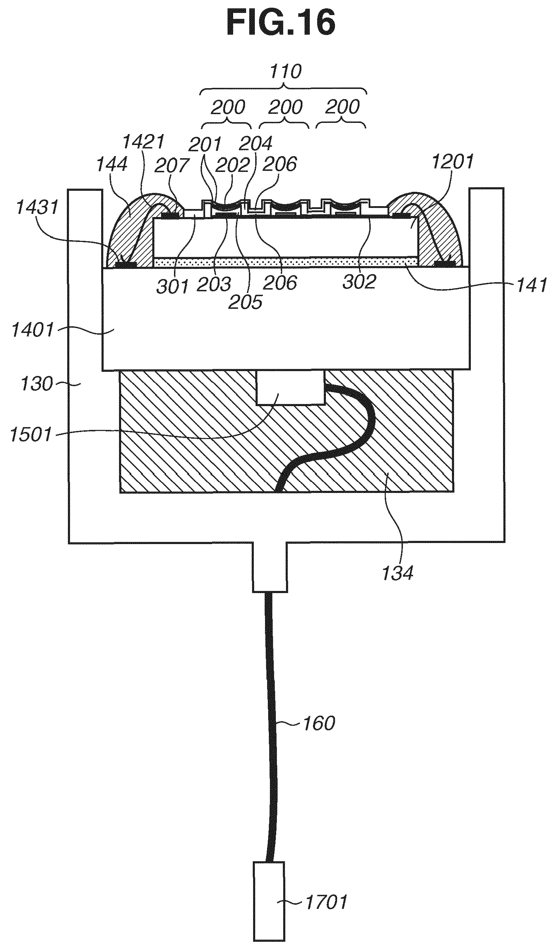

Fig. 16 is a schematic view illustrating a photoacoustic wave probe 100 according to the present exemplary embodiment.

According to the present exemplary this embodiment, a space surrounded by an interposer 1401 and a housing portion 130 in the transducer unit 103, and a portion for drawing out a cable 160 is filled with potting material 134. The potting material 134 can be a material which is used for insulation and moisture-proofing in an electronic device. A material, such as an urethane-based resin and an epoxy-based resin, can be used. According to the present exemplary embodiment, a wiring portion for connecting a CMUT 110 to the cable 160 can be perfectly insulated from the outside of the transducer unit 103. Further, since moisture outside the transducer unit 103 is prevented from entering the wiring portion, failure of the wiring due to humidity is unlikely to occur and reliability of the wiring can be enhanced. Further, when this configuration is combined with the configuration of the tenth or eleventh embodiment, failure in a detecting circuit 402 can be reduced.

Further, when the configuration according to the present exemplary embodiment is combined with the configuration of the thirteenth exemplary embodiment, the transducer unit 103 with significantly high reliability can be provided.

A fifteenth exemplary embodiment relates to a shape of a member for supporting a chip 1201. As for others, this embodiment is the same as any of the ninth to fourteenth embodiments.

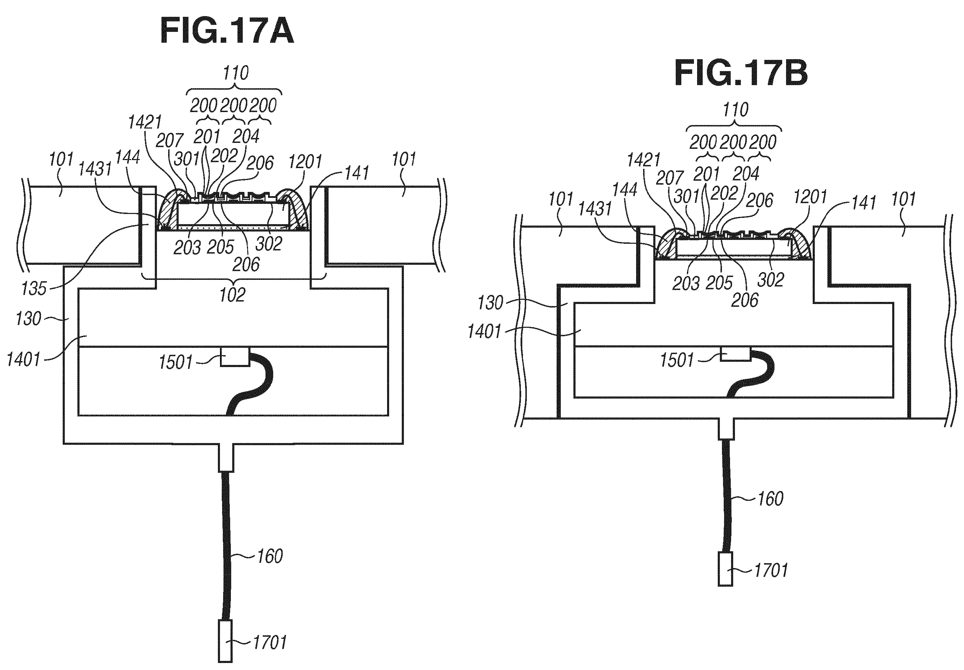

Fig. 17A and 17B are schematic cross sectional views each illustrating a transducer unit 103 in a photoacoustic wave probe 100 according to the present exemplary embodiment.

According to the present exemplary embodiment, as illustrated in Fig. 17A, an interposer 1401 includes a protrusion 144. On the protrusion 144 of the interposer 1401, the chip 1201 with a CMUT 110 is arranged. A housing portion 130 according to the present exemplary embodiment has a convex shape corresponding to a shape of the interposer 1401 having the protrusion 144.

The thickness of the hemispherical support member 101 is less than the height of a protrusion 135 of the housing portion 130, so that when the transducer unit 103 is inserted in the support member 101, only the protrusion 135 of the housing portion 130 is inserted in a through hole 102. Further, portions of the transducer unit 103 other than the protrusion 135 is arranged projecting outside the support member 101. Thus, according to the present exemplary embodiment, only the protrusion 135 in which the chip 1201 of the transducer unit 103 is disposed is small in its diameter, and the diameter of the through hole 102 in the support member 101 can be preferably made small. Hence, mechanical strength of the support member 101 can be enhanced. Therefore, the hemispherical support member 101 is unlikely to bend, and the positional relationship between arranged transducer units 103 is maintained with high accuracy while a photoacoustic wave is received. Hence, it is possible to provide the photoacoustic wave probe 100 which can accurately acquire information of a photoacoustic wave from the object.

Further, in the interposer 1401 according to the present exemplary embodiment, a shape of the protrusion 135 supporting the chip 1201 and a shape of the rear side for disposing a detecting circuit 402 thereon can be changed. Therefore, the size of the protrusion 135 can be most suitably minimized for the arrangement of the chip 1201, and, as described above, the mechanical strength of the hemispherical support member 101 can be enhanced. On the other hand, the region of wiring for arrangement of the detecting circuit 402 is not influenced by restriction in size of the through hole 102 and necessary area can be ensured. Thus, the wiring can be configured most suitably for characteristics. Therefore, the CMUT 110 with excellent reception characteristics can be provided.

According to the present exemplary embodiment, the through hole 102 in the support member 101 is formed such that the transducer unit 103 is arranged with portions other than the protrusion 135 projected outside the support member 101. However, the configuration is not limited thereto. For example, as illustrated in Fig. 17B, the support member 101 can have a concave portion (a counterbore) corresponding to the shape of the transducer unit 103, in addition to the through hole 102 of the support member 101. Accordingly, even when the thickness of the support member 101 is larger than the height of the protrusion of the transducer unit 103, the transducer unit 103 can be suitably arranged. Therefore, the mechanical strength of the support member 101 can be further enhanced.

A sixteenth exemplary embodiment relates to a shape of a member for supporting a chip 1201. As for others, the present exemplary embodiment is the same as fifteenth embodiment.

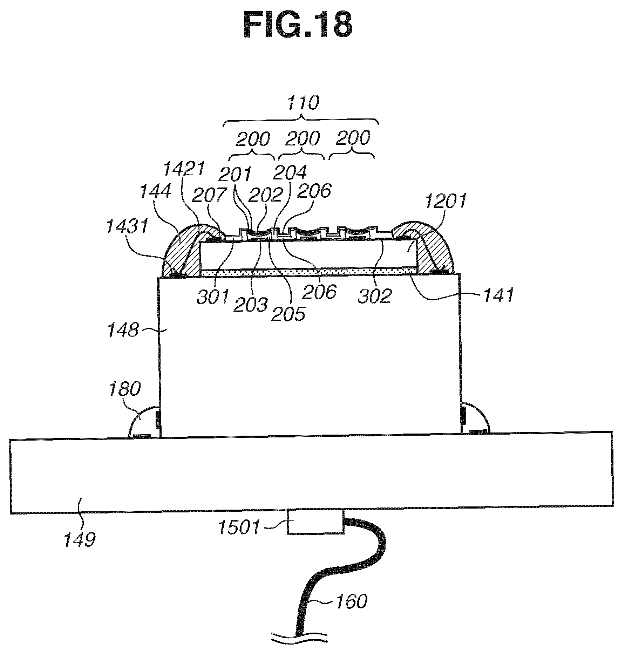

Fig. 18 is a schematic cross sectional view illustrating a transducer unit 103 in a photoacoustic wave probe 100 according to the present exemplary embodiment. In Fig. 18, the housing portion 130 is omitted. According to the present embodiment, the interposer 1401 with the protrusion 135 for supporting the chip 1201, illustrated in the fifteenth exemplary embodiment, includes a first interposer 148 and a second interposer 149. According to the present exemplary embodiment, the first interposer 148 is a molded interconnect device (MID) in which wiring is formed on a surface of a molded product, and the second interposer 149 is a multilayer circuit substrate including glass epoxy and a copper thin layer.

On the first interposer 148, a component with a desired shape is molded and wiring can be easily formed on a surface of the component, using the MID technology. Therefore, wiring from the chip 1201 can be easily drawn out close to the second interposer 149. The wiring of the first interposer 148 can be electrically connected to an electrode on the second interposer 149 with solder 180 easily.

Since the first interposer 148 is formed by molding, a high protrusion can be easily fabricated. Therefore, the protrusion of the transducer unit 103 can be easily formed higher, and even when the support member 101 is thickened, the transducer unit 103 can be arranged without interference of its lower portion. Thus, the hemispherical support member 101 can be thickened and is unlikely to bend, and a photoacoustic wave can be received while a positional relationship between arranged transducer units 103 is maintained with high accuracy. Therefore, is it possible to provide the photoacoustic wave probe which can accurately acquire information of a photoacoustic wave from the object.

A seventeenth exemplary embodiment relates to a shape of a through hole 102. As for others, the present exemplary embodiment is the same as any of the ninth to sixteenth embodiments, and accordingly description thereof is omitted.

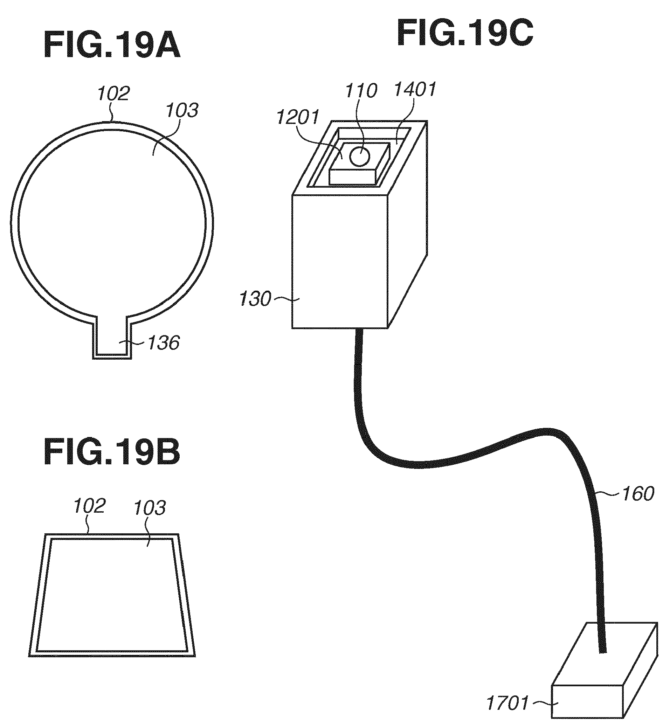

Fig. 19A to 19C are schematic views each illustrating an acoustic wave transducer unit 103 disposed in a photoacoustic wave probe 100 according to the present exemplary embodiment. Fig. 19A and 19B are schematic top views each illustrating the transducer unit 103 installed in the through hole 102 disposed in the support member 101 and a relationship between an inner wall of the through hole 102 and an outward form of the transducer unit 103.

According to the present exemplary embodiment, as illustrated in Fig. 19A, a side face of a housing portion 130 in the transducer unit 103 is provided with a protrusion 136 for alignment with the support member 101. Further, the through hole 102 in the support member 101 has a shape corresponding to the protrusion 136. According to the present exemplary embodiment, even when a position of a CMUT 110 shifts from a center part relative to an outward form of the transducer unit 103 viewed from the center of the hemisphere of the photoacoustic wave probe 100, the position of the CMUT 110 in the hemispherical support member 101 can be accurately set.

According to the present exemplary embodiment, it is possible to provide the photoacoustic wave probe 100 in which the CMUT 110 can be arranged at a predetermined position even when the position of the CMUT 110 shifts from the center part of the transducer unit 103.

Another example of the present exemplary embodiment will be described referring to Fig. 19B. In Fig. 19B, a shape of a cross section of the through hole 102 is trapezoidal. Corresponding to the shape of the through hole 102, a shape of a cross section of the housing portion 130 of transducer unit 103 is also trapezoidal. Since cross sections of the through hole 102 and the transducer unit 103 are trapezoidal, the ultrasonic transducer unit 103 can be prevented from being positioned in a direction different from a predetermined direction when the ultrasonic transducer unit 103 is fixed to the support member 101. Therefore, in the configuration in which the CMUT 110 is arranged in a position shifted from the center of the transducer unit 103, each transducer unit 103 can be fixed without failure when the transducer unit 103 is arranged in the support member 101. Therefore, it is possible to provide the photoacoustic wave probe 100 in which assemblage problem of the transducer unit 103 is unlikely to occur, and an assemblage operation can be efficiently executed.

Yet another example of the present exemplary embodiment will be described referring to Fig. 19C. The configuration in Fig. 19C is the same as that of the fifteenth embodiment except that the cross sectional shape of the through hole 102 is rectangular. The outward form of the protrusion of the transducer unit 103 corresponding to the cross sectional shape of the through hole 102 is quadrangular, and the outward form of the first interposer 148 in the inner portion of the transducer unit 103 is also quadrangular. A quadrangular shape is the most easily manufactured shape of the chip 1201 on which the CMUT 110 is arranged. Therefore, with the configuration illustrated in Fig. 19C, the protrusion of the transducer unit 103 can be minimized. Since the through hole 102 corresponding to the transducer unit 103 can be minimized, the mechanical strength of the support member 101 can be enhanced.

An eighteenth exemplary embodiment relates to a detecting circuit 402 connected to a CMUT 110 on a chip 1201. As for others, the present exemplary embodiment is the same as the tenth to seventeenth exemplary embodiments.

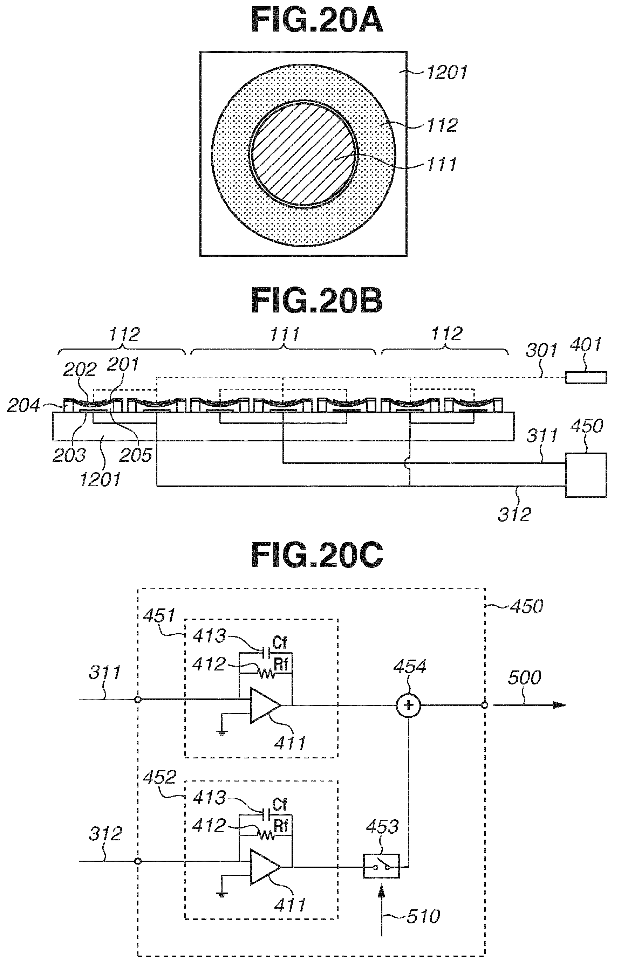

Fig. 20A is a top schematic view illustrating a transducer unit 103 in a photoacoustic wave probe 100 according to the present exemplary embodiment. Fig. 20B is a cross sectional view illustrating a portion of a CMUT 110 in the transducer unit 103.

Fig. 20C is a circuit diagram illustrating a circuit in the photoacoustic wave probe 100 according to the present exemplary embodiment.

According to the present exemplary embodiment, a plurality of first CMUTs 111 and a plurality of second CMUTs 112 are arranged on the chip 1201, and a plurality of detecting circuits 451 and 452 are connected to the first and second CMUTs 111 and 112, respectively. As illustrated in Figs. 20A and 20B, there are disposed a plurality of groups on the chip 1201 in each of which first electrodes 202 are electrically connected to each other. A region of the first CMUTs 111 is circular viewed along a directional axis and is surrounded by a donut-shaped region of the second CMUTs 112.

Meanwhile, as illustrated in Fig. 20C, a plurality of detecting circuits 451 and 452 are disposed on an interposer 1401. The first CMUT 111 is connected to the detecting circuit 451, and the second CMUT 112 is connected to the detecting circuit 452. Output voltage of the detecting circuit 452 is connected to an adder 454 through a switch 453. In the adder 454, output voltage of the detecting circuit 451 is added to voltage from the switch 453. The element size of CMUTs which is used to receive an acoustic wave can be readily changed by changing the switch 453 between a connect condition and a non-connect condition.

When a photoacoustic wave from the object is received, the most suitable size of CMUTs differs. Directionality of reception of an acoustic wave (ultrasound) differs depending on the size of CMUTs, so that it is preferable to change the size of transducers used for reception, i.e., the number thereof, according to information to be acquired from the object. When the number of transducers 110 is increased to widen a detection region, a low frequency band can be readily received. In contrast, when the number of transducers 110 is decreased to narrow down the detection region, a high frequency band can be readily received. According to the present exemplary embodiment, since the size of CMUTs for receiving a photoacoustic wave can be readily changed, the directionality of a transducer can be changed depending on the object whose information is to be acquired, or the purpose. Thus, the most desired information of the object can be obtained.

A nineteenth exemplary embodiment relates to a generating unit of DC voltage to be applied to a CMUT 110. As for others, this embodiment is the same as any of the ninth to eighteenth exemplary embodiments, and accordingly, description thereof is omitted.

In Fig. 21A there is provided an applied voltage adjusting unit 460. According to the present exemplary embodiment, between a DC voltage generating unit 401 and a second electrode 203, the adjusting unit 460 for adjusting applied voltage is disposed. The adjusting unit 460 has a function of adjusting Vb output from the DC voltage generating unit 401 to Vo at a terminal applied to the second electrode 203.

In the CMUT 110, depending on the variation in thickness of a vibration film 201 and the variation of a gap 205, a most suitable value of DC voltage to be applied to each element differs. In the ultrasonic probe 100 according to the present exemplary embodiment, the most suitable DC voltage Vo is applied to each element of the CMUT 110 including a plurality of cells. Referring to Fig. 21B, a structure of a circuit of the adjusting unit 460 for the applied voltage will be described.

The adjusting unit 460 for adjusting applied voltage includes three voltage dividing resistors. A first resistor 461 is inserted between the DC voltage generating unit 401 and the second electrode 203. A second resistor 462 and a third resistor 463 are connected in series, and are arranged between a second wiring 302 on a side of the second electrode 203 and a ground (GND) terminal. Where a value of the first resistor 461 is R1, a value of the second resistor 462 is R2, and a value of the third resistor 463 is R3, a value of voltage Vo applied to the wiring on the side of the second electrode 203 can be represented by the following formula:

$$\mathrm{Vo} = (\mathrm{R2+R3})/(\mathrm{R1+R2+R3}) * \mathrm{Vb}.$$

Thus, the applied voltage is adjustable. Therefore, different voltage most stable for each element can be applied to the CMUT 110.

According to the present exemplary embodiment, a value of R2 is made smaller than a value of R3. Thereby, a voltage drop at the second resistor 462 is smaller than a voltage drop at the third resistor 463. Therefore, the first resistor 461 and the third resistor 463 need to be high withstand voltage (several tens voltages to several hundred voltages) devices. In contrast thereto, a lower withstand voltage device can be used for the second resistor 462. Since the first resistor 461 and the third resistor 463 are high withstand voltage resistors, large components need to be used. However, a small-sized component can be used as the second resistor 462.

With the presence of the second resistor 462, even when values of the first resistor 461 and the third resistor 463 are fixed, the applied voltage can be changed only by changing the value of the second resistor 462. The second resistor 462 only needs to be set to such a value that applied voltage corresponding to each element can be achieved. Since the second resistor 462 is a small-sized component, replacement thereof is easy.

Further, a first high withstand voltage capacitor 464 and a second high withstand voltage capacitor 465 are arranged for purposes of suppressing variation in voltage at each terminal and inflow of noise from outside.

In the photoacoustic wave probe 100 according to the present exemplary embodiment, since the most suitable DC voltage can be applied to each element, characteristics of the CMUT 110 can be made uniform. Hence, information can be acquired with high accuracy. Therefore, when the photoacoustic wave probe 100 according to the present exemplary embodiment is used, an image with high image quality can be acquired.

According to the present exemplary embodiment, the first resistor 461 and the third resistor 463 are arranged in a body 999 of an apparatus. The second resistor 462 is arranged on an interposer 1401. The value of the second resistor 462 is set to a resistance value that generates applied voltage corresponding to each element, and a different value is used for each element. Therefore, even when the transducer unit 103 disposed in the support member 101 is changed, the most suitable applied voltage can be set for each element without changing a resistance value in the body 999 of the apparatus. Further, withstand voltage of the second resistor 462 is small and a small-sized component is used as the second resistor 462. Accordingly, the second resistor 462 can be arranged in a small mounting region on the interposer 1401.

Wiring connecting the support member 101 to a flexible portion includes third wiring 303 connected to the second resistor 462, in addition to first wiring 301 connected to a first electrode 202 of the CMUT 110 and second wiring 302 connected to a second electrode 203 of the CMUT 110. Hence, the voltage Vo applied to a capacitive transducer can be adjusted by the second resistor 462 disposed on the interposer 1401. In this configuration, three (3) resistors are used, but at least two resistors will do.

In the probe provided with a plurality of ultrasonic transducers on the curved surface, according to the present exemplary embodiment, it is possible to provide a probe characterized in that the configuration of wiring for connecting each element to the outside can be simplified and reception frequency characteristics of the ultrasonic transducer are uniform and excellent.

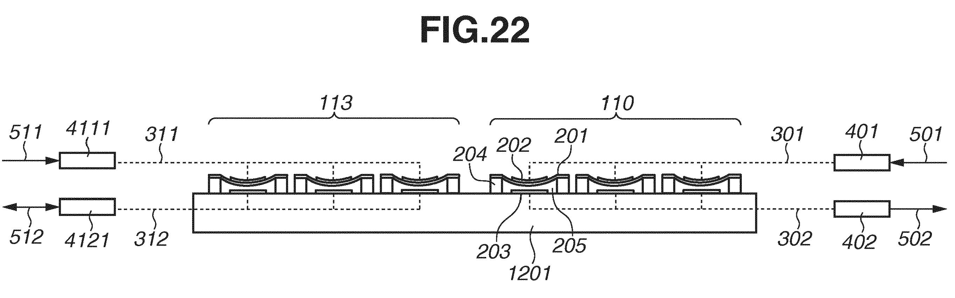

In a twentieth exemplary embodiment, a CMUT 113 for transmitting ultrasound is provided in addition to a CMUT 110 for receiving photoacoustic wave. As for others, the present exemplary embodiment is the same as the seventeenth to nineteenth exemplary embodiments. Fig. 22 illustrates the present exemplary embodiment. In FIG.22, there are provided a bias voltage 511 and a ultrasonic transmitting and receiving signal 512.

In Fig. 22, there are provided a CMUT 113 for transmitting and receiving ultrasound, a second DC voltage applying unit 4111, and a driving circuit 4121. Fig. 22 is a schematic diagram illustrating an element of the CMUTs 110 for reception and an element of the CMUTs 113 for transmission arranged on a chip 1201. The CMUT 113 has characteristics suitable for transmitting ultrasound. A second DC voltage applying unit 4111 is adjusted to apply voltage such that ultrasound transmission efficiency becomes the most suitable. Further, the driving circuit 4121 can apply high voltage pulses to a second electrode 203. A first electrode 202 of the CMUT 113 on each chip is electrically connected to the driving circuit 4121. Hence, electrostatic attractive force generated between the first electrode 202 and the second electrode 203 is changed by the driving circuit 4121, so that a vibration film 201 is vibrated to transmit ultrasound.

On the other hand, the CMUT 110 has the structure suitable for reception of ultrasound as well as reception of a photoacoustic wave. Hence, when the CMUT 110 receives a photoacoustic wave and ultrasound, the vibration film 201 is vibrated and a detecting circuit 402 detects the vibration.

According to the present exemplary embodiment, at the time of forming an ultrasound image, the CMUT 113 for transmission transmits ultrasound toward the object, and the CMUT 110 for reception receives ultrasound reflected in the object. At the time of acquiring a photoacoustic image, the CMUT 110 for reception receives a photoacoustic wave generated in the object irradiated with light from a light source 106.

With the photoacoustic wave probe 100 according to the present exemplary embodiment, both reception of a photoacoustic wave and transmission and reception of ultrasound can be executed using a single probe. Therefore, a photoacoustic image and an ultrasound image can be formed based on detected information. Further, the CMUT 113 suitable for ultrasound transmission is separately provided from the CMUT 110 suitable for reception of ultrasound and a photoacoustic wave. Therefore, both of excellent characteristics of ultrasound transmission and excellent reception characteristics can be satisfied, an image with high quality can be acquired. Further, the CMUTs 113 and 110 used for transmission and reception of ultrasound and reception of a photoacoustic wave are arranged on the same chip 1201. Accordingly, an image can be obtained with little shift between a photoacoustic image and an ultrasound image.

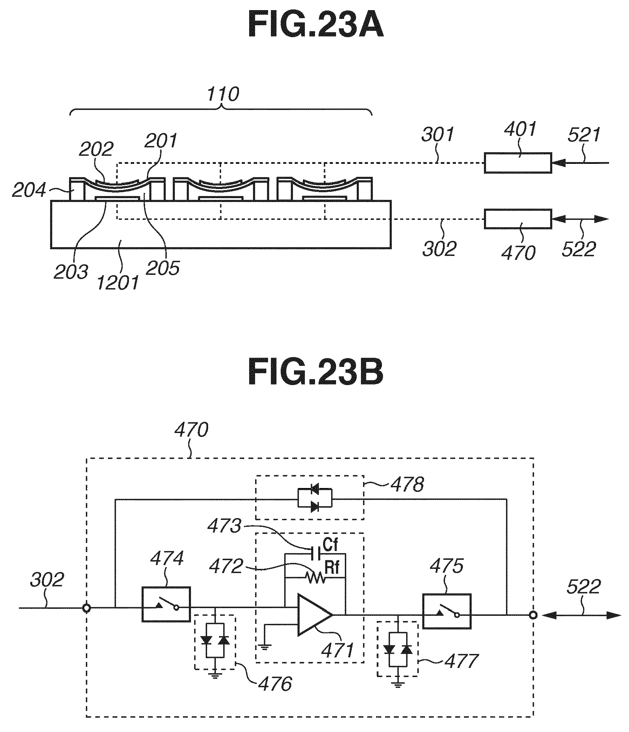

In a twenty-first exemplary embodiment, a CMUT 110 has a function of performing transmission and reception of ultrasound. As for others, the present exemplary embodiment is the same as the ninth to twentieth embodiments. Figs. 23A and 23B illustrates the present exemplary embodiment.

In Fig. 23B, a driving detecting circuit 470 includes an operation amplifier 471, a feedback resistor 472, a feedback capacitor 473, high withstand voltage switches 474 and 475, diodes 476 and 477, and a high withstand voltage diode 478. Fig. 23A is a schematic view illustrating the CMUT 110 arranged on a chip 1201. On each chip 1201, an element of the CMUTs 110 is arranged, and a first electrode 202 of the CMUT 110 is connected to a detecting circuit 451. The detecting circuit 451 applies to the CMUT 110 high voltage pulses for use of ultrasound transmission supplied from an apparatus, and outputs a minute current from the CMUT 110 to the apparatus as a detected signal.

Fig. 23B is a circuit diagram illustrating a circuit of another example of the detecting circuit 451 in Fig. 20C. A feedback resistor 472 and a feedback capacitor 473 are arranged in parallel in a negative feedback portion of the operation amplifier 471, and the function of current-voltage conversion is executed. To input and output terminals of the operation amplifier 471, the high withstand voltage switches 474 and 475 and the diodes 476 and 477 are respectively connected. When a voltage between terminals is below a predetermined voltage (1 or less than one (1) voltage), the high withstand voltage diode 478 disconnects the wiring connection between the terminals. Further, when voltage above a predetermined voltage (about several voltages) is applied, the high withstand voltage switches 474 and 475 disconnect a wiring connection between input and output terminals.

When no high voltage pulse for transmission is applied, the high withstand voltage diode 478 disconnects the wiring connection between input and output terminals because little electric potential difference is present between the terminals. On the other hand, since high voltage is not applied from outside, the high withstand voltage switches 474 and 475 execute the wiring connection between the switches. Therefore, minute current from the transducer 110 is converted to voltage by the operation amplifier 471, and a detected signal can be supplied to the apparatus (not shown) connected to the outside.

In contrast, when a high voltage pulse for transmission is applied from the apparatus (not shown), wiring in an inner portion of the high withstand voltage diode 478 is connected, and high voltage above predetermined voltage (about several voltages) is applied to the high withstand voltage switches 474 and 475. Since the high withstand voltage switches 474 and 475 can disconnect wiring connections in inner portions of the high withstand voltage switches 474 and 475, high voltage is unlikely to be applied to the operation amplifier 471 and break down of the operation amplifier 471 is unlikely to occur. Since a signal output from the operation amplifier 471 is stopped at the high withstand voltage switch 475, the signal output gives no influence on a high voltage pulse for transmission. Therefore, it is possible to apply a high voltage pulse for transmitting ultrasound to the first electrode 202 in the transducer 110.

With the photoacoustic wave probe 100 according to the present exemplary embodiment, both of reception of a photoacoustic wave and transmission and reception of ultrasound can be executed using a single probe. Therefore, a photoacoustic image and an ultrasound image can be formed based on detected information. Further, since the CMUT 110 can perform both of transmission of ultrasound and reception of ultrasound and a photoacoustic wave, the size of the chip 1201 can be reduced. Therefore, cells of the CMUTs 110 can be arranged closer to each other, and the number of elements can be increased. Alternatively, in a case where the same number of elements is arranged a hemispherical probe with a small diameter can be realized. Further, since the CMUT 110 is used in common, an image can be obtained with less positional shift between a photoacoustic image and an ultrasound image.

A twenty-second exemplary embodiment relates to a detecting circuit 402 connected to a CMUT 110 on a chip 1201. As for others, the present exemplary embodiment is the same as the tenth to seventeenth exemplary embodiments.

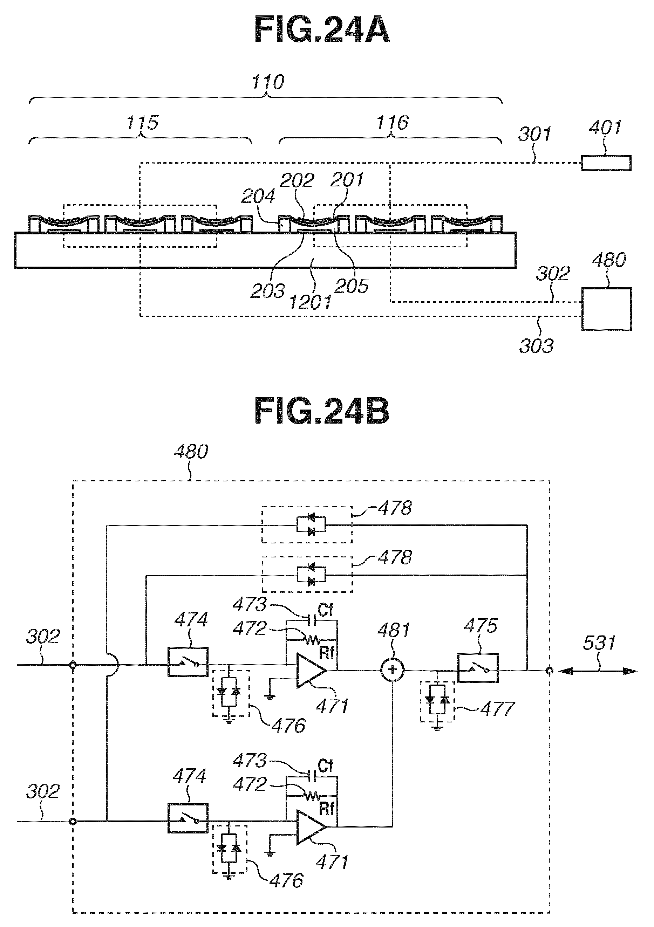

Fig. 24A is a schematic enlarged view illustrating a portion of the CMUT 110 in the photoacoustic wave probe 100 according to the present exemplary embodiment. Fig. 24B is a circuit diagram illustrating a driving detecting circuit 480 that is another example of the detecting circuit 402 in the tenth exemplary embodiment. According to the present exemplary embodiment, a plurality of CMUTs 115 and 116 are arranged on a chip 1201. More specifically, there arranged is a plurality of groups on the chip 1201, in each of which second electrodes 203 in the CMUT 110 are electrically connected. Wirings from the second electrodes 203 in respective groups are connected to different input terminals of the driving detecting circuit 480 arranged on an interposer 1401. At the time of transmission of ultrasound, high voltage driving signals from an external apparatus are applied from each terminal to first electrodes 202 of the CMUT 110, through a plurality of transmission diodes in the driving detecting circuit 480.

At the time of reception, signals from the second electrodes 203 are each amplified by operation amplifiers 471, and each of the operation amplifiers 471 outputs voltage. Each of the output voltages is added up at an adder 481 and output through wiring (not shown) in a cable 160.

The maximum value of a capacitor capable of being connected to an input terminal of each operation amplifier 471 is determined depending on sizes of a resistor and a capacitor in a feedback portion, and gain frequency characteristics of the operation amplifier 471. According to the present exemplary embodiment, since the CMUT 110 is arranged in separate groups and each operation amplifier 471 is connected to a different group, capacitance of the CMUT 110 connected to each operation amplifier 471 can be reduced. Thereby, it is possible to most suitably set parameters of the feedback portion in the operation amplifier 471, widen a receiving frequency band, and make better use of characteristics of the CMUT 110 with a broad band.

Further, in a transimpedance circuit using the operation amplifier 471, an output noise changes depending on a capacitor connected to the input terminal of the operation amplifier 471. According to the present exemplary embodiment, since the CMUT 110 is arranged in a separate manner and each operation amplifier 471 is connected to a different group of the CMUT 110, an output noise can be reduced in each current-voltage converting circuit. In a case where an output from each operation amplifier 471 is added by the adder 481, an output noise can be reduced to about $1/\sqrt{X}$ where X is the division number. Hence, as a whole, an output noise can be reduced, and a received signal can be detected with high accuracy.

When the circuit according to the present exemplary embodiment as illustrated in Fig. 24B is used, ultrasound transmission and reception by the CMUT can be executed without a control signal from outside. According to such a configuration, it is possible to provide the photoacoustic wave probe characterized in that frequency characteristics of current-voltage conversion at the time of reception are excellent, a noise in an output signal is little, and a receiving operation is excellent.

In a twenty-third exemplary embodiment, the number of cells for ultrasound transmission and the number of cells for reception of a photoacoustic wave and ultrasound can be changed. As for others, the present exemplary embodiment is the same as the twenty-first exemplary embodiment.

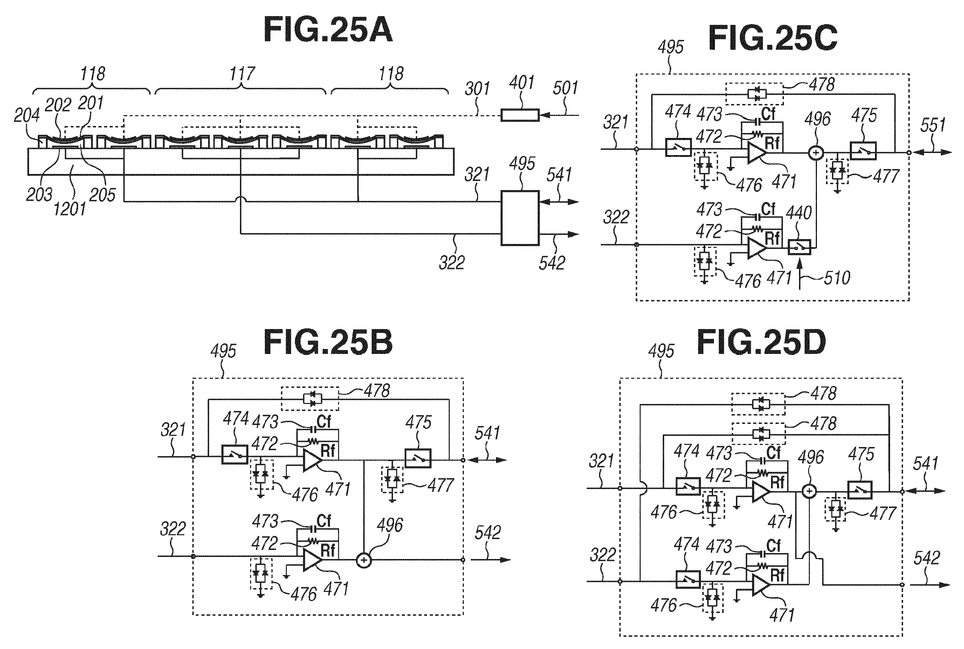

As illustrated in Fig. 25A, there are arranged first CMUTs 117 for transmission and reception of ultrasound, and second CMUTs 118 for reception, on a chip 1201.

A second electrode 203 in the first CMUT 117 is connected to a first terminal 491 in a driving detecting circuit 495. A second electrode 203 in the second CMUT 118 is connected to a second terminal in the driving detecting circuit 495. In the driving detecting circuit 495, in addition to the circuit described in the eighteenth exemplary embodiment, there are arranged a current-voltage converting circuit (transimpedance circuit) using an operation amplifier 471, and an adder 496. Hereinafter, difference from the twenty-first exemplary embodiment will be described.

Fig. 25B is a circuit diagram illustrating the driving detecting circuit 495 in the photoacoustic wave probe 100 according to the present exemplary embodiment. When a photoacoustic wave is received, a high withstand voltage diode 478 is in an OFF state and front and rear wirings of the operation amplifier 471 are in an ON state. Accordingly, current from the first CMUT 117 is converted to voltage. A signal 542 is used as a received signal. The adder 496 forms the signal 542 by adding up a voltage signal of the first CMUT 117 and a received signal formed by conversion of current from the second CMUT 118 to voltage. Thereby, ultrasound can be received by both of the first CMUT 117 and the second CMUT 118.

At the time of transmission and reception of ultrasound, a driving signal 541 for ultrasound is input to the driving detecting circuit 495. When high voltage is applied, the operation amplifier 471 is protected from high voltage, and the high withstand voltage diode 478 is in the ON state. Therefore, driving voltage is applied only to the first CMUT 117 connected to wiring 321, and ultrasound is transmitted from the vibration film. When a vibration film 201 is vibrated by ultrasound reflected in the object (measurement object), the high withstand voltage diode 478 is in the OFF state because the driving signal 541 for transmission (driving) of ultrasound is not applied. Then, since the front and rear wirings of the operation amplifier 471 are in the ON state, current-voltage conversion is performed and the signal 542 of reception of ultrasound is output.

In the photoacoustic wave probe 100 according to the present exemplary embodiment, it is possible to change the size of an element used for reception of a photoacoustic wave and the size of an element used for reception and transmission of ultrasound. Therefore, reception of a photoacoustic wave and reception and transmission of ultrasound can be executed using elements whose sizes are suitable for acquisition of each signal. Therefore, it is possible to acquire information to be needed to generate a photoacoustic image and an ultrasound image with high image quality.

Further, according to the present exemplary embodiment, the number of cells of the first CMUTs 117 and the second CMUTs 118 used for reception of a photoacoustic wave is made larger than the number of cells of the first CMUTs 117 used for ultrasound transmission. At the time of reception of a photoacoustic wave, the element size is large, so that directionality of a received photoacoustic wave can be enhanced in a case where the same frequency is used. On the other hand, at the time of transmission and reception of ultrasound, multiplication of the directionality of an element at transmission and the directionality of an element at reception works even when an element with a small diameter is used. Therefore, compared to an element with the same diameter executing only reception, it is possible to increase the directionality after transmission and reception. Therefore, when the photoacoustic wave probe 100 according to the present exemplary embodiment is used, it is possible to obtain an image in which resolutions of a photoacoustic image and an ultrasound image are closer to each other.

As another example of the present exemplary embodiment, a driving detecting circuit 495 illustrated in Fig. 25C can be used. According to this example, the driving detecting circuit 495 includes a switch 440 between the operation amplifier 471 connected to the second CMUT 118 and the adder 496. The switch 440 is ON/OFF controlled by a control signal 510 of the switch 440. More specifically, the control signal 510 is input such that the switch 440 is in an OFF state at the time of transmission and reception of ultrasound, and in an ON state at the time of reception of a photoacoustic wave. The switch 440 can be easily realized using a low voltage analog switch.

According to the present exemplary embodiment, signals of transmission and reception of ultrasound and an output signal of a photoacoustic wave can be transmitted through a single wiring (signal 551), and the number of wirings in the cable of the probe can be reduced. The control signal 510 for the switch 440 is not necessarily input from an external apparatus. When application of a high voltage pulse of a driving signal to the photoacoustic wave probe 100 is detected, and in a case where a unit for generating an OFF-signal for the switch 440 for a certain period of time is provided, there is no need of inputting a signal from outside.

Fig. 25D illustrates yet another example of the present exemplary embodiment. According to this example, only the first CMUT 117 is used for reception of a photoacoustic wave, and the first CMUT 117 and the second CMUT 118 are used for transmission and reception of ultrasound.

It is therefore possible to enlarge the size of an element for transmission and reception, transmit larger ultrasound, and enhance sensitivity of reception. Therefore, transmission and reception signals with high accuracy can be acquired. According to the present exemplary embodiment, center frequency of ultrasound used for transmission and reception is set larger than the center frequency of a reception signal of a photoacoustic wave. Thereby, it is possible to obtain an image characterized in that resolutions of an photoacoustic image and an ultrasound image become closer to each other and an ultrasound image can be acquired with higher accuracy.

Any of the photoacoustic wave (ultrasonic) probes according to the ninth to twenty-third exemplary embodiments can be used to receive a photoacoustic wave (ultrasound) due to the photoacoustic effect, and applied to a photoacoustic apparatus (object information acquisition apparatus) including any of the photoacoustic wave (ultrasonic) probes. A twenty-fourth embodiment relates to such apparatuses.

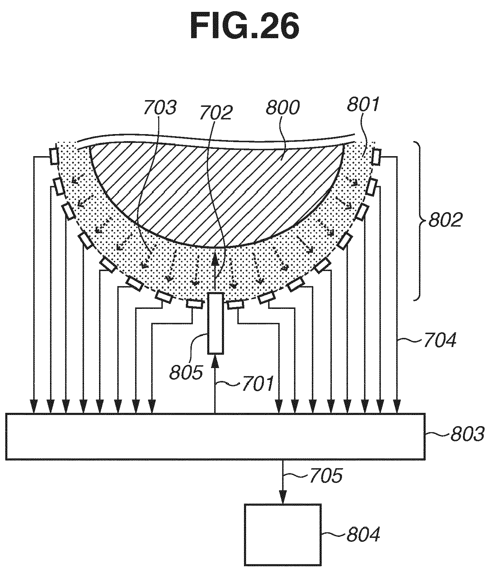

Referring to Fig. 26, an operation of an ultrasonic measurement apparatus according to the present exemplary embodiment will be specifically described. Based on a signal 701 of a light emission instruction, light 702 (pulse light) is emitted from a light source 805, and a measurement object 800 is irradiated with the light 702. In the measurement object 800, a photoacoustic wave (ultrasound) 703 is generated due to irradiation with the light 702, and the ultrasound 703 is received by a plurality of capacitive transducers 802 in the ultrasonic probe. Information of the received wave, such as size, shape, and time, is transmitted as the reception signal 704 of the photoacoustic wave to an image information generating apparatus 803. Meanwhile, information (light emission information) of the light 702 generated in the light source 805, such as size, shape, time of the light 702, is stored in a storing portion (not illustrated) in the image information generating apparatus 803. In the image information generating apparatus 803, an image signal of the measurement object 800 is generated based on the photoacoustic wave reception signal 704 and information of the light 702 emitted from the light source 805, and reproduction image information 705 generated from the photoacoustic signal is output. In an image display unit 804, an image of the measurement object 800 is displayed based on the reproduction image information 705 generated from the photoacoustic signal.

The ultrasonic measurement apparatus according to the present exemplary embodiment has a simple structure and has characteristics that a photoacoustic wave in a broad frequency range can be received is realized. Therefore, much information can be acquired from a photoacoustic wave using a small-sized probe, the apparatus can be easily handled, and an image with high image quality can be generated.

In a twenty-fifth exemplary embodiment, the photoacoustic wave probe in any of the twentieth to twenty-third exemplary embodiments capable of transmitting and receiving ultrasound is applied to the photoacoustic apparatus of the twenty-fourth exemplary embodiment.

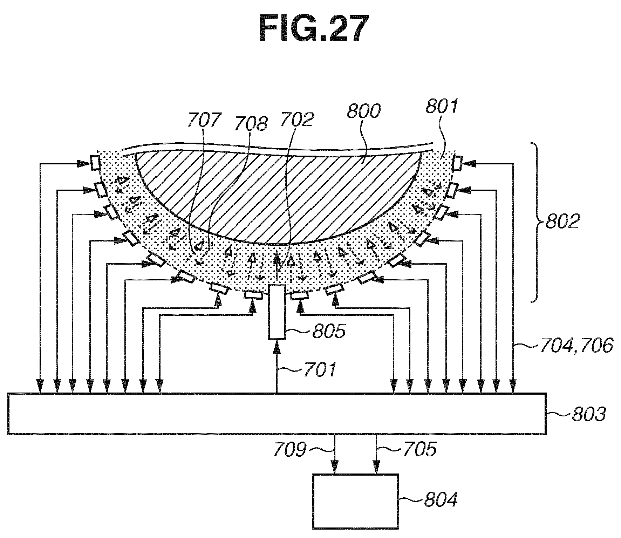

Fig. 27 is a schematic diagram illustrating a photoacoustic apparatus according to the present exemplary embodiment. In Fig. 27, there provided a transmission and reception signal 706 of ultrasound, transmitted ultrasound 707, reflected ultrasound 708 from the object, and reproduction image information 709 due to transmission and reception of ultrasound.

In the photoacoustic apparatus according to the present exemplary embodiment, in addition to reception of a photoacoustic wave, pulse echo (transmission and reception of ultrasound) is performed to form an image. Reception of the photoacoustic wave according to the present exemplary embodiment is the same as in the twentieth exemplary embodiment. Here, only pulse echo (transmission and reception of ultrasound) will be described.

Based on the transmission signal 706 of ultrasound, ultrasound 707 is transmitted from a plurality of capacitive transducers 802 toward a measurement object 800. In an inner portion of the measurement object 800, ultrasound is reflected due to a difference in intrinsic acoustic impedance of a substance present inside the measurement object 800. The reflected ultrasound 708 is received by a plurality of the capacitive transducers 802, and information of a received signal, such as size, shape, and time, is transmitted to an image information generating apparatus 803 as a reception signal 706 of ultrasound. Meanwhile, information of the transmitted ultrasound, such as size, shape, and time, is stored in a storing portion in the image information generating apparatus 803 as ultrasound transmission information. In the image information generating apparatus 803, an image signal of the measurement object 800 is generated based on the reception signal 706 of ultrasound and the ultrasound transmission information, and reproduction image information 709 generated by ultrasound transmission and reception is output.

In an image display unit 804, an image of the measurement object 800 is displayed based on two pieces of information of reproduction image information 705 based on a photoacoustic signal and the reproduction image information 709 produced from transmission and reception of ultrasound.

According to the present exemplary embodiment, the ultrasonic probe capable of transmitting and receiving a photoacoustic wave in a broad frequency range is used, and an image is formed from pieces of information each acquired by a different type of measurement method. Therefore, it is possible to acquire and display an image having a large amount of information.

According to the present exemplary embodiments, the first electrode 202 is disposed on the vibration film 201, and the second electrode 203 is disposed on the substrate 120. However, the configuration is not limited thereto. Alternatively, the second electrode 203 can be formed on the vibration film 201 and the first electrode 202 can be formed on the substrate 120.

According to the present exemplary embodiments, it is possible to provide an acoustic wave probe and so forth characterized in that deterioration of characteristics of an acoustic wave transducer unit including one or more transducers due to installation to a support member is unlikely to occur.

With the photoacoustic wave probe and the photoacoustic apparatus including the photoacoustic wave probe according to another example of the present exemplary embodiments, it is possible to suppress occurrence of turbulent flow of acoustic matching material and temperature distribution of the acoustic matching material in association with the turbulent flow, using a simple structure.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.

**Claims**

1. An acoustic wave probe comprising:

   a support member including a plurality of through holes and a concave portion, the concave portion being concave facing an object disposed in a measurement position at the time of measurement; and
acoustic wave transducer means including at least a transducer,

wherein the acoustic wave transducer means is mounted in the through hole approximately facing a center of curvature of the concave portion, and wherein a thickness of the acoustic wave transducer means is thinner on a side of the center of curvature.

2. The acoustic wave probe according to claim 1, wherein in the acoustic wave transducer means, an area of a cross section of an end portion on a side of the center of curvature is smaller than an area of a cross section of another end portion on a side opposite to the side of the center of curvature, and an area of cross section of a portion between the end portion and the another end portion is equal to or smaller than the area of the cross section of the another end portion.

3. The acoustic wave probe according to claim 1 or 2, wherein the concave portion of the support member is approximately spherical.

4. The acoustic wave probe according to any of claims 1 to 3, wherein an alignment mechanism is provided between the support member and the acoustic wave transducer means.

5. The acoustic wave probe according to claim 4, wherein the alignment mechanism is a fitting mechanism comprising a set of a portion in an outward shape of the acoustic wave transducer means and a portion in an inside shape of the through hole corresponding to the portion in the outward shape of the acoustic wave transducer means.

6. The acoustic wave probe according to claim 5, wherein the alignment mechanism is a fitting mechanism comprising a set of a protrusion portion and a concave portion.

7. The acoustic wave probe according to claim 5, wherein the alignment mechanism is a fitting mechanism comprising a set of a polygon shape and a polygon shape.

8. The acoustic wave probe according to claim 4, wherein the alignment mechanism is a fitting mechanism comprising a set of a pin and an aperture, one of which is formed in one of a peripheral portion of the through hole in the support member and a flange portion of the acoustic wave transducer means, and the other of which is formed in the other of the peripheral portion and the flange portion.

**9.** The acoustic wave probe according to any of claims 1 to 8, wherein a sealing member is provided between the acoustic wave transducer means and the support member such that acoustic matching material is prevented from entering a gap between the acoustic wave transducer means and the support member.

**10.** The acoustic wave probe according to any of claims 1 to 9, wherein the acoustic wave transducer means includes a transducer for executing at least one of conversion of an acoustic wave to a reception signal and conversion of a transmission signal to an acoustic wave, the transducer being disposed on an end portion on the side of the center of curvature.

**11.** The acoustic wave probe according to claim 10, wherein the transducer is a capacitive electromechanical transducer.

**12.** The acoustic wave probe according to any of claims 1 to 11, further comprising:

a light irradiation portion for irradiating the object with light therefrom, the light irradiation portion being mounted to the support member.

**13.** Acoustic wave transducer means comprising:

at least a transducer disposed on an end portion of the acoustic wave transducer means, wherein an area of a cross section of an end portion is smaller than an area of a cross section of another end portion on a side opposite to the side of the end portion, and an area of a cross section of a portion between the end portion and the another portion is equal to or smaller than the area of the cross section of the another end portion.

**14.** The acoustic wave transducer means according to claim 13, wherein a shape of the cross section of the end portion is polygonal.

**15.** An object information acquisition apparatus comprising:

the acoustic wave prove recited in any of claims 1 to 12, wherein the acoustic wave prove receives a photoacoustic wave generated in an object due to photoacoustic effect, and acquires information of the object.

**16.** An object information acquisition apparatus comprising:

the acoustic wave prove recited in any of claims 1 to 12, wherein the acoustic wave prove receives a photoacoustic wave generated in an object due to photoacoustic effect and transmits ultrasound to the object and receives the ultrasound from the object to acquire information of the object.

**17.** A photoacoustic wave probe comprising:

a plurality of transducer means each including a transducer for receiving a photoacoustic wave generated due to photoacoustic effect in an object irradiated with light from a light source, and outputting an electric signal; and
a support member for supporting the plurality of transducer means such that directional axes of transducers gather together,

wherein the plurality of transducer means is disposed in apertures formed in the support member, and the plurality of transducer means is arranged such that the plurality of transducer means does not project from the support member toward a side on which the directional axes gather together.

**18.** The photoacoustic wave probe according to claim 17, wherein the shape of the support member is hemispherical, and surfaces of the plurality of transducer units on a side on which the directional axes gather together are arranged along a curved surface of the hemispherical shape.

**19.** The photoacoustic wave probe according to claim 17 or 18, wherein the transducer is a capacitive transducer.

# FIG.1A

100

103

101

104

# FIG.1B

101

103

102

106

160

104

# FIG.1C

110

120

130

103

142

142

140

X

Y

Z

160

# FIG.1D

102

101

110

101

151

150

103

150

151

140

# FIG.2A

110

120

130

103

142

142

140

143

X

Y

Z

160

# FIG.2B

101 102 110 101

151 150 103 150 151 140

# FIG.2C

101 102 110 101

143 150 103 150 140

152

# FIG.3A

# FIG.3B

# FIG.3C

# FIG.3D

# FIG.4A

# FIG.4B

# FIG.5A

110
120
121
133
130
103
1321
160

X
Y
Z

# FIG.5B

Z
Z1
Y
X

121
110
101
102
120
101
133
130
132
150
170
150

# FIG.5C

101
102
103
130
133

# FIG.5D

110
120
121
1321
133
130
103
160

X
Y
Z

# FIG.5E

# FIG.5F

# FIG.5G

## FIG.6A

110
121
120
130
103
1321

X
Y
Z

160

## FIG.6B

Z
Z2

Y
X

101 121 102 110 120 101

130
132

150 170 150

## FIG.6C

101
102
103
130

## FIG.6D

110
121
120
130
103
1321

X
Y
Z

160

# FIG.7

# FIG.8

# FIG.9

# FIG.10A

# FIG.10B

# FIG.10D

# FIG.10C

# FIG.10E

# FIG.11A

# FIG.11B

# FIG.12A

# FIG.12B

# FIG.13A

# FIG.13C

# FIG.13B

# FIG.13D

EP 3 135 190 A1

# FIG.14

**FIG.15A**

**FIG.15B**

# FIG.16

# FIG.17A

# FIG.17B

EP 3 135 190 A1

# FIG.18

# FIG.19A

# FIG.19C

*102*

*103*

*136*

*1201*

*110* *1401*

*130*

*160*

*1701*

# FIG.19B

*102* *103*

# FIG.20A

# FIG.20B

# FIG.20C

# FIG.21A

# FIG.21B

# FIG.22

EP 3 135 190 A1

# FIG.23A

# FIG.23B

EP 3 135 190 A1

# FIG.24A

# FIG.24B

48

FIG.25A

FIG.25B

FIG.25C

FIG.25D

EP 3 135 190 A1

# FIG.26

# FIG.27

**EUROPEAN SEARCH REPORT**

Application Number

EP 16 18 5288

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 6 216 025 B1 (KRUGER ROBERT A [US]) 10 April 2001 (2001-04-10) | 1-5,10, 13,15-18 | INV. A61B5/00 |
| Y | * abstract; figures 1-3 * <br> * column 3, line 14 - line 30 * <br> * column 4, line 23 - column 6, line 21 * <br> * column 7, line 41 - column 8, line 18 * <br> ----- | 7,11,12, 14,19 | A61B8/00 |
| X | US 5 437 194 A (LYNNWORTH LAWRENCE C [US]) 1 August 1995 (1995-08-01) <br> * abstract; figures 1,2,4 * <br> * column 2, line 9 - line 35 * <br> * column 6, line 41 - column 7, line 7 * <br> * column 7, line 31 - line 52 * <br> ----- | 1,3-6,8, 9 | |
| Y | US 2015/114125 A1 (TANAKA TAKATOSHI [JP] ET AL) 30 April 2015 (2015-04-30) <br> * paragraphs [0030], [0031], [0040] - [0043]; figure 2 * <br> ----- | 11,12,19 | |
| Y | EP 2 823 897 A2 (CANON KK [JP]) 14 January 2015 (2015-01-14) <br> * paragraph [0016]; figures 2a,2b * <br> ----- | 7,14 | TECHNICAL FIELDS SEARCHED (IPC) |
| A | JP 2003 203953 A (TOSHIBA CORP) 18 July 2003 (2003-07-18) <br> * abstract; figure 2 * <br> ----- | 1-6,8,13 | A61B <br> B06B <br> G10K <br> G01S <br> G01N |
| A | US 2012/285250 A1 (RHIM SUNG MIN [KR] ET AL) 15 November 2012 (2012-11-15) <br> * paragraphs [0007] - [0017], [0021], [0023], [0024], [0035], [0038] - [0039]; figures 1-5 * <br> ----- | 17 | |
| A | US 2006/103265 A1 (MIYOSHI TETSU [JP]) 18 May 2006 (2006-05-18) <br> * abstract; figures 21,1a-1c * <br> ----- | 1,13 | |

The present search report has been drawn up for all claims

| Place of search | Date of completion of the search | Examiner |
|---|---|---|
| Munich | 19 January 2017 | Daoukou, Eleni |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 16 18 5288

This annex lists the patent family members relating to the patent documents cited in the above-mentioned European search report.
The members are as contained in the European Patent Office EDP file on
The European Patent Office is in no way liable for these particulars which are merely given for the purpose of information.

19-01-2017

| Patent document cited in search report | | Publication date | Patent family member(s) | | Publication date |
|---|---|---|---|---|---|
| US 6216025 | B1 | 10-04-2001 | AU | 2866700 A | 25-08-2000 |
| | | | BR | 0007738 A | 06-11-2001 |
| | | | CA | 2361076 A1 | 10-08-2000 |
| | | | EP | 1158905 A2 | 05-12-2001 |
| | | | JP | 2002536041 A | 29-10-2002 |
| | | | NO | 20013598 A | 01-10-2001 |
| | | | US | 6216025 B1 | 10-04-2001 |
| | | | WO | 0045707 A2 | 10-08-2000 |
| US 5437194 | A | 01-08-1995 | EP | 0746764 A1 | 11-12-1996 |
| | | | JP | 3715647 B2 | 09-11-2005 |
| | | | JP | H09508202 A | 19-08-1997 |
| | | | US | 5437194 A | 01-08-1995 |
| | | | WO | 9519559 A2 | 20-07-1995 |
| US 2015114125 | A1 | 30-04-2015 | JP | 2015085200 A | 07-05-2015 |
| | | | US | 2015114125 A1 | 30-04-2015 |
| EP 2823897 | A2 | 14-01-2015 | CN | 104281772 A | 14-01-2015 |
| | | | EP | 2823897 A2 | 14-01-2015 |
| | | | JP | 5855050 B2 | 09-02-2016 |
| | | | JP | 2015019224 A | 29-01-2015 |
| | | | KR | 20150007245 A | 20-01-2015 |
| | | | US | 2015016221 A1 | 15-01-2015 |
| JP 2003203953 | A | 18-07-2003 | JP | 3692080 B2 | 07-09-2005 |
| | | | JP | 2003203953 A | 18-07-2003 |
| US 2012285250 | A1 | 15-11-2012 | EP | 2527828 A1 | 28-11-2012 |
| | | | JP | 5542966 B2 | 09-07-2014 |
| | | | JP | 2013517088 A | 16-05-2013 |
| | | | KR | 20110084802 A | 26-07-2011 |
| | | | US | 2012285250 A1 | 15-11-2012 |
| | | | WO | 2011087192 A1 | 21-07-2011 |
| US 2006103265 | A1 | 18-05-2006 | JP | 4601471 B2 | 22-12-2010 |
| | | | JP | 2006158939 A | 22-06-2006 |
| | | | US | 2006103265 A1 | 18-05-2006 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20110306865 A